# EUROPEAN PATENT SPECIFICATION

(11) **EP 2 171 089 B1**
(45) Date of publication and mention of the grant of the patent: **22.01.2014**
(21) Application number: 08773655.9
(22) Date of filing: 25.06.2008
(51) Int. Cl.: C12Q 1/68, A61K 38/08

(54) **SCREENING-METHOD FOR POLYMORPHIC MARKERS IN HTRA1 GENE IN NEURODEGENERATIVE DISORDERS**
SCREENING-VERFAHREN FÜR POLYMORPHE MARKER IM HTRA1-GEN BEI NEURODEGENERATIVEN STÖRUNGEN
PROCÉDÉ DE CRIBLAGE POUR DES MARQUEURS POLYMORPHES DANS LE GÈNE HTRA1 DANS DES TROUBLES NEURODÉGÉNÉRATIFS

(30) Priority: 25.06.2007 EP 07012418; 25.06.2007 US 946020 P; 08.04.2008 EP 08006972
(43) Date of publication of application: 07.04.2010
(73) Proprietor: Universität Duisburg-Essen, 45141 Essen (DE)
(72) Inventor: EHRMANN, Michael, 45138 Essen (DE); EGENSPERGER, Rupert, 81243 München (DE); TENNSTADT, Annette, 50670 Köln (DE)
(74) Representative: Roth, Carla
(86) International application number: PCT/EP2008/005150
(87) International publication number: WO 2009/000520

(56) References cited:
- EP-A- 0 828 003
- WO-A-2004/108899
- WO-A-2008/013893
- US-A1- 2004 014 109
- US-A1- 2005 164 933
- YANG ZHENGLIN ET AL: "A variant of the HTRA1 gene increases susceptibility to age-related macular degeneration." SCIENCE (NEW YORK, N.Y.) 10 NOV 2006, vol. 314, no. 5801, 10 November 2006 (2006-11-10), pages 992-993, XP002460077 ISSN: 1095-9203
- CORDER E H ET AL: "GENE DOSE OF APOLIPOPROTEIN E TYPE 4 ALLELE AND THE RISK OF ALZHEIMER'S DISEASE IN LATE ONSET FAMILIES" SCIENCE, AMERICAN ASSOCIATION FOR THE ADVANCEMENT OF SCIENCE,, US, vol. 261, 13 August 1993 (1993-08-13), pages 921-923, XP000619582 ISSN: 0036-8075
- RUNYON STEVEN T ET AL: "Structural and functional analysis of the PDZ domains of human HtrA1 and HtrA3" PROTEIN SCIENCE, CAMBRIDGE UNIVERSITY PRESS, CAMBRIDGE, GB, vol. 16, no. 11, 1 November 2007 (2007-11-01), pages 2454-2471, XP009103036 ISSN: 0961-8368
- MA SUCAN ET AL: "Rapid characterization of the binding property of HtrA2/Omi PDZ domain by validation screening of PDZ ligand library" SCIENCE IN CHINA. SERIE C: LIFE SCIENCE, GORDON AND BREACH, AMSTERDAM, NL, vol. 50, no. 2, 1 June 2007 (2007-06-01), pages 412-422, XP009103037 ISSN: 1006-9305
- GRAU SANDRA ET AL: "Implications of the serine protease HtrA1 in amyloid precursor protein processing." PROCEEDINGS OF THE NATIONAL ACADEMY OF SCIENCES OF THE UNITED STATES OF AMERICA 26 APR 2005, vol. 102, no. 17, 26 April 2005 (2005-04-26), pages 6021-6026, XP002497611 ISSN: 0027-8424
- CHURCHER IAN: "TAU THERAPEUTIC STRATEGIES FOR THE TREATMENT OF ALZHEIMER'S DISEASE" CURRENT TOPICS IN MEDICINAL CHEMISTRY, BENTHAM SCIENCE PUBLISHERS, HILVERSUM, NL, vol. 6, no. 6, 1 January 2006 (2006-01-01), pages 579-595, XP009081772 ISSN: 1568-0266
- OZTURK A ET AL: "Three SNPs in the GSTO1, GSTO2 and PRSS11 genes on chromosome 10 are not associated with age-at-onset of Alzheimer's disease" NEUROBIOLOGY OF AGING, TARRYTOWN, NY, US, vol. 26, no. 8, August 2005 (2005-08), pages 1161-1165, XP004904944 ISSN: 0197-4580
- "Copaxone in Age Related Macular Degeneration"[Online] April 2007 (2007-04), pages 1-5, XP002497612 Retrieved from the Internet: URL:http://clinicaltrials.gov/ct2/show/NCT 00466076> [retrieved on 2008-09-23]
- MURWANTOKO ET AL: "Binding of proteins to the PDZ domain regulates proteolytic activity of HtrA1 serine protease." THE BIOCHEMICAL JOURNAL 1 AUG 2004, vol. 381, no. Pt 3, 1 August 2004 (2004-08-01), pages 895-904, XP002497613 ISSN: 1470-8728

## Description

The present invention relates to methods of screening, in particular diagnosing and prognosing neurodegenerative diseases such as in particular Alzheimer's disease (AD) wherein a genetic risk factor for said neurodegenerative disease is determined. Furthermore, therapeutic approaches for treating neurodegenerative diseases such as AD are provided.

The HtrA (high temperature requirement) family represents a new class of oligomeric serine proteases the activity of which is regulated by reversible zymogen activation. The defining feature of the over 180 family members is the combination of a catalytic domain with one or more C-terminal PDZ domains. PDZ domains are protein modules that mediate specific protein-protein interactions and bind preferentially to the C-terminal 3-4 residues of the target protein. Prokaryotic HtrAs have been attributed to the tolerance against various folding stresses as well as to pathogenicity. The four human homologues are believed to be involved in arthritis, cell growth, unfolded protein response, cancer, ageing, placental development and function, Parkinson disease and in metabolism of amyloid precursor protein (Abou-Sleiman et al., Nat Rev Neurosci 7:207-219 (2006); Clausen et al., Mol Cell 10, 443-455 (2002); Grau et al., Proc. Natl. Acad. Sci. USA 102:6021-6026 (2005); Nie et al., Placenta 27:491-501 (2006).

The human family members can be divided into two groups. HtrA2 possesses a transmembrane anchor and a large section of the N-terminus can be removed by processing. The N-termini of HtrAs 1, 3 and 4 all contain predicted signal peptides as well as sections that are recognized as insulin growth factor binding protein (IGFBP) and protease inhibitor domains. Initial evidence suggests that stress response pathways might be involved in their regulation.

The HtrA1 gene (PRSS11) was initially identified as being expressed in human fibroblasts but not after transformation with SV40 (Zumbrunn and Trueb, FEBS Lett 398:187-92 (1996)).

Recent studies indicate that PRSS11 mRNA is either absent or significantly downregulated in ovarian cancer (Shridhar et al., Cancer Res 62: 262-270 (2002)), leukaemia, Burkitt's lymphoma and melanomas (Baldi et al., Oncogene 21:6684-6688 (2002)). In addition, overexpression of HtrA1 inhibited proliferation *in vitro* and tumor growth *in vivo* (Baldi et al., Oncogene 21:6684-6688 (2002)). These results suggest a tumor suppressor function. A tumor suppressor phenotype of a protease is interesting as so far this function was mainly attributed to protease inhibitors. HtrA1 is mainly secreted but a subfraction is also localised in the cytosol (Grau et al., J Biol Chem 281:6124-6129 (2006)). Secreted HtrA1 is likely to be involved in the degradation of extracellular matrix proteins and APP fragments (Grau et al., Proc. Natl. Acad. Sci. USA 102:6021-6026 (2005)).

Age-related macular degeneration (AMD) is the most common cause of irreversible vision loss among the ageing population throughout the developed world. It is classified as either wet (neovascular) or dry (nonneovascular). While inherited variation in the complement factor H gene was found to be a major risk factor for drusen in dry AMD (Hageman et al., Proc. Natl. Acad. Sci. USA 102:7227-32 (2005)), it was recently reported that a single nucleotide polymorphism (rs11200638) in the promoter region of HtrA1 is a major genetic risk factor for wet AMD. A whole-genome association mapping strategy was applied to a Chinese population. Individuals with the risk-associated genotype were estimated to have a likelihood of developing wet AMD 10 times that of individuals with the wild-type genotype (Dewan et al., Science 314:989-992 (2006); Yang et al., Science 314:992-993 (2006)). Additionally preliminary analysis of lymphocytes and retinal pigment epithelium from Caucasian wet AMD patients revealed that the risk allele was associated with elevated expression levels of HtrA1 mRNA and protein. Furthermore, drusen from the eyes of AMD patients strongly immunolabelled with HtrA1 antibodies (Yang et al., Science 314:992-993 (2006).

A neurodegenerative disease is a condition in which in particular cells of the brain and/or spinal cord are lost. The brain and spinal cord are composed of neurons having different functions such as controlling movements, processing sensory information, and making decisions. Neurodegenerative diseases usually result from deterioration of neurons which over time will lead to neurodegeneration and disabilities resulting from this. In particular, neurodegenerative diseases cause problems with movements or affect the memory such as conditions related to dementia. Neurodegenerative diseases have multiple causes and risk factors, thereunder protein folding defects leading to protein or protein fragment aggregation. These protein folding or amyloid diseases include e.g. Prion disease such as Creutzfeldt Jacob disease, Huntington's disease, Tauopathies, and alpha-Synucleinopathies such as Parkinson's disease and Lewy body dementia. Many times neuronal death begins long before the patient will ever experience any symptoms. Hence, an early diagnosis of a neurodegenerative disease or a risk factor for it is important.

Dementias such as Alzheimer's disease (AD) are characterised by the occurrence of various pathological features including the formation of neurofibrillary tangles within neurons, neuronal loss, reactive gliosis, inflammation and the accumulation of amyloid ß (Aß) in the walls of blood vessels and senile plaques (Selkoe Physiol. Rev. 81:741-766 (2001)). The hallmark of AD is the occurrence of protein and peptide aggregates ultimately leading to the formation of senile plaques in patient brains.

Intracellular neurofibrillary tangles (NFT) and neuritic plaques contain tau, a microtubule associated protein that aggregates following hyperphosphorylation (Avila, FEBS Lett. 80:2922-2927 (2006)). The other major constituents of neuritic plaques are Aß peptides that are generated by interplay of various secretases cleaving amyloid precursor protein (APP) and C99. They accumulate in the brain and form senile plaques mainly consisting of two Aß species, Aß40 and Aß42 (Haass, et al. Nature 359: 322-325 (1992), Seubert et al. Nature 361:260-263 (1993)). In addition, tau is sequestered into glial tangles in astrocytes or oligodendroglia.

Normal tau protein, which is abundant in axons, is associated with the stabilisation of microtubules of the cytoskeleton. Interaction with microtubules is mediated by so called tubulin binding repeats (Mandelkow et al., Brain Pathol 17:83-90 (2007)). Posttranslational modifications, such as phosphorylation can modulate the affinity of tau to microtubules (Mazanetz et al. Nature Rev Drug Discov 6:464-479 (2007)). Upon hyperphosphorylation tau disassociates from microtubules and polymerises forming straight or paired helical filaments (PHF), ribbons and other conformations (Buee et al. Brain Res. Rev. 33, 95-130 (2000)). Progressive tau polymerisation leads to the formation of NFT which parallels with the loss of synapses in the frontal cortex. Tau in PHFs is proteolytically processed into a core domain of three repeats that are involved in stable tau-tau interactions (Jakes et al. EMBO J. 10:2725-2729 (1991)). PHF tau aggregates can further capture and provide a template for processing of full-length tau proteins (Wischik et al. Proc. Natl. Acad. Sci. USA 93:11213-11218 (1996)). This mechanism leads to progressive growth of the toxic protein aggregates eventually causing cell death. Cell death correlates with formation of extracellular NFTs that are immunoreactive against tau. Early studies reporting a correlation of the total level of NFTs and the degree of cognitive impairment and thus the importance of tau for disease development are supported by in vivo studies where reduction or elimination of endogenous tau reduces Aß mediated deficits (Arriagada et al. Neurology 42: 631-639 (1992); Arriagada et al. Neurology 42:1681-1688 (1992); Roberson et al. Science 316:750-754 (2007)).

While some proteases are described in the literature that might be involved in tau processing, these experiments were mainly done in vitro and most proteases are unable to degrade tau or hyperphosphorylated tau completely (Sengupta et al. Biochemistry 45:15111-9 (2006); Kenessey et al. J Neurochem 69:2026-38 (1997); Guillozet-Bongaarts et al. J Neurochem 97:1005-14 (2006)). Especially, the proteolytic degradation of the repeats that mediate tau-tau interaction and therefore its aggregation into tangles would be of great therapeutic interest. However, a protease performing this function is unknown. In addition, the proteases identified so far failed to proteolyse hyperphosphorylated tau. Furthermore, a protease able to cleave aggregates of normal or hyperphosphorylated tau remains to be found. Therefore, there is a great need to identify the so far elusive tau protease that is associated with AD and other tauopathies.

Accordingly, AD is characterised by the occurrence of various pathological features including the formation of neurofibrillary tangles within neurons, neuronal loss, reactive gliosis, inflammation and the accumulation of amyloid ß (Aß) in the walls of blood vessels and senile plaques (Selkoe Physiol. Rev. 81:741-766 (2001). Aß peptides are generated by interplay of various secretases cleaving amyloid precursor protein (APP) and C99. They accumulate in the brain and form senile plaques mainly consisting of two Aß species, Aß40 and Aß42. Although Aß peptides are continuously secreted from cells (Haass, et al. Nature 359: 322-325 (1992), Seubert et al. Nature 361:260-263 (1993)), a metabolic balance prevents plaque formation. An imbalance in Aß levels, caused for example by mutations in various AD related genes, results in the accumulation and aggregation of these peptides (Czech et al. Prog. Neurobiol. 60:363-384 (2000); Hutton, M. & Hardy, J. Hum. Mol. Genet. 6:1639-1646 (1997)). Clearance of cerebral Aß peptides can be achieved either by excretion into blood through the blood-brain barrier, microglia or degradation by proteolytic enzymes (Tanzi, et al. Neuron 43: 605-608 (2004)). Several candidate proteases such as insulin degrading enzyme (IDE), neprilysin, endothelin converting enzyme and HtrA1 have been implicated in the removal of Aß (Tanzi, et al. Neuron 43: 605-608 (2004); Grau et al. Proc. Natl. Acad. Sci. USA 102:6021-6026 (2005)). The other major constituent of neuritic plaques is tau protein, a microtubule associated protein that aggregates following hyperphosphorylation (Avila, FEBS Lett. 80:2922-2927 (2006).

While rare (3% of all AD cases) familial, autosomal dominant, early onset AD is associated with mutations in the amyloid precursor protein (APP) (Goate et al., Nature 349:704-706 (1991)), and presenilin (PSEN1 (Sherrington et al., Nature 375:754-760 (1995)) and PSEN2 (Levy-Lahad et al., Science 269:973-977 (1995); Rogaev et al., Nature 376:775-778 (1995)) genes, 95% of AD patients are classified as late-onset cases.

The genetic basis of late-onset AD (LOAD) is complex, with the involvement of multiple genes and various environmental factors. The apolipoprotein E (APOE) gene located on chromosome 19q13.2 is the only well established risk factor for AD that has been consistently replicated as a risk factor for LOAD, and that is associated with both risk and age at onset (AAO) in late-onset familial AD, as well as in late- and early-onset sporadic AD (Saunders et al., Neurology 43:1467-1472 (1993); Schmechel et al., Proc. Natl. Acad. Sci. USA 90: 9649-9653 (1993); Strittmatter et al., Proc. Natl. Acad. Sci. USA 90:1977-1981 (1993); (Corder et al., Science 261:921-923 (1993)). However, about 50% of AD patients do not carry the APOE-epsilon 4 allele, and only 30% of AD patients and <10% of the variance in AAO appear to be explained by the APOE-epsilon 4 allele (Slooter et al., Arch. Neurol. 55:964-968 (1998); Daw et al., Am. J. Hum. Genet. 66:196-204 (2000)). Further risk factors are thus involved.

Genome-wide linkage studies also suggest the existence of multiple additional genes for LOAD on several chromosomes, including chromosome 10. A broad linkage peak encompassing a >50 mb region between chromosome 10q21 and 10q25 has been implicated as influencing either AD risk or plasma levels of Aß42 (Bertram et al., Science 290:2302-2303 (2000); Blacker et al., Hum. Mol. Genet. 12:23-32 (2003); Myers et al., Science 290:2304-2305 (2000); Myers et al., Am. J. Med. Genet. 114: 235-244 (2002)).

Several candidate genes near the chromosome 10 linkage peaks were implicated in LOAD, including the catenin (cadherin-associated protein) alpha 3 (CTNNA3) (Mirra et al., Neurology 41:479-486 (1991), insulin-degrading enzyme (IDE), urokinase plasminogen activator (PLAU) and the glutathione S-transferase omega-1 and 2 (GSTO1 and GSTO2) genes (Bertram et al., Hum Mol Genet 13 Spec No 1:R135-141 (2004)). However, the association results of these candidate genes have not been consistently replicated.

In addition to the disease risk, AAO of AD is also genetically controlled and one of the AAO genes has been mapped on the distal end of chromosome 10 that overlaps with the linkage peak of AD risk or Aß42 levels (Li, et al., Am. J. Hum. Genet. 70:985-993 (2002)). Using differential gene expression approach in brains from AD patients and controls, (Li, et al., Am. J. Hum. Genet. 70:985-993 (2002)) found that 4 of the 52 differentially expressed genes (stearoyl-COA desaturase; NADH ubiquinone oxidoreductase 1ß complex 8; PRSS11 (HtrA1); and glutathione *S*-transferase, omega 1 or GSTO1) were located under the linkage peak for AAO on chromosome 10. Thus, these genes were targeted for association analysis using a large family-based sample. Two of the four differentially expressed genes, PRSS11 (HtrA1) and GSTO1, and a linked member of the GST omega class, GSTO2, showed significant association with AAO in both AD and Parkinson Disease. A recent study however, suggested that three SNPs in the GSTO1, GSTO2 and PRSS11 (HtrA1) genes on chromosome 10 are not associated with AAO of AD. (Ozturk et al., Neurobiol Aging 26:1161-1165 (2005).

At present, the only well established genetic risk factor for LOAD is the APOE-epsilon 4 allele. However, about 50% of AD patients do not carry the APOE-epsilon 4 allele, and only 30% of AD patients and <10% of the variance in AAO appear to be explained by the APOE-epsilon 4 allele (Slooter et al., Arch. Neurol. 55:964-968 (1998); Daw et al., Am. J. Hum. Genet. 66:196-204 (2000)). In addition, ApoE4 has only mild effects on the onset of the disease and the plaque load of patients. Even though genetic tests for the ApoE4 allele are available (see e.g. US5508167), there is no reliable biological method for diagnosing AD in patients before autopsy or without obtaining cerebrospinal fluid. In addition, none of the available therapies are able to combat the disorder in the mid or long-term. Therefore, identification of additional factors that increase the risk to develop AD or causing an earlier onset of the disease are required.

The identification of further genes involved in the risk or developing AD or other neurodegenerative diseases such as Parkinson's disease would open new avenues of research with the potential to delay onset beyond the natural life span. Present knowledge about genes contributing to AAO in neurodegenerative diseases clearly lags behind the understanding of genes contributing to the development of the risk. Recently, there has been growing interest in using AAO information as a quantitative trait, to identify genes that influence onset of disease (Daw et al., Am J Hum Genet 64:839-851 (1999), Daw et al., Am J Hum Genet 66:196-204 (2000); Duggirala et al., Am J Hum Genet 64:1127-1140 (1999)). Nevertheless, the genetic basis of neurodegenerative diseases such as AD or Parkinson Disease is not well understood, and there is a continued need to identify new functional polymorphisms that are associated with neurodegenerative diseases and in particular the AAO.

Therefore, it is the object of the invention to provide methods for screening risk factors associated with neurodegenerative diseases, in particular AD. Furthermore, it is the object of the present invention to provide therapeutic approaches for treating/preventing neurodegenerative diseases such as AD. Furthermore, it is the object of the present invention to identify the tau protease that is associated with AD and other tauopathies.

According to one embodiment, the problem is solved by a method of screening a subject for at least one risk factor associated with a tauopathy, in particular AD (Alzheimer's disease), comprising detecting the presence or absence of at least one risk marker in the HtrA1 gene (PRSS11) which is linked to said tauopathy, wherein the risk marker is a polymorphism or mutation of the HtrA1 gene.

According to one central aspect, the inventors have found that the long sought risk factor located on chromosome 10q associated with AD is the HtrA1 gene. Genetic variations in the HtrA1 gene cause an earlier onset of AD. The term "gene" also includes regulatory elements such as e.g. promoter and enhancer regions. The effects of allelic variations in the HtrA1 gene are even stronger than those of the established risk factor ApoE. Also the life span of subjects carrying certain allelic variations in the HtrA1 gene is reduced. Thus, the HtrA1 gene is a valuable target marker for determining the risk that a subject is at risk of developing AD. The screening method according to the present invention thus characterises the HtrA1 genotype of individuals for diagnosing AD, prognosing the risk for developing AD or prognosing/determining AAO of AD.

However, the present method is not only suitable for detecting a risk factor of AD as the HtrA1 gene is also a risk factor for other neurodegenerative diseases, namely tauopathies. Important risk factors for neurodegenerative diseases are protein folding defects leading to protein or protein fragment aggregation. These protein folding or amyloid diseases include e.g. the following diseases: Prion diseases such as Creutzfeldt Jacob disease; Huntington's disease; Tauopathies which are a group of diverse dementias and movement disorders which have as a common pathological feature the presence of intracellular accumulations of abnormal filaments of tau protein; Alpha-Synucleinopathies which describe a group of disorders having in common the abnormal deposition of alpha-synuclein in the cytoplasm of neurons or glial cells, as well as in extracellular deposits of amyloid. In Parkinson's disease and Lewy body dementia, alpha-synuclein is the main component of Lewy bodies and dystrophic neurites; alpha-synuclein also accumulates in the cytoplasm of glial cells.

The inventors have found that HtrA1 is involved in the processing of key players of neurodegenerative diseases, in particular amyloid precursor fragments and tau. Proteomics data also indicate that apolipoproteins such as ApoE are substrates of HtrA1. Hence, the HtrA1 gene is a target and thus risk marker for neurodegenerative diseases in general.

The detecting step may include detecting whether the subject is heterozygous or homozygous for the risk marker such as a functional polymorphism in the HtrA1 gene, with subjects who are at least heterozygous for the functional polymorphism being at increased risk for developing a neurodegenerative disease, in particular AD. The step of detecting the presence or absence of the risk marker such as a functional polymorphism may thus include the step of detecting the presence or absence of the marker such as a functional polymorphism in both chromosomes of the subject (i.e., detecting the presence or absence of one or two alleles containing the marker or functional polymorphism). More than one copy of a marker or functional polymorphism (i.e., subjects homozygous for the functional polymorphism) may indicate greater risk of developing a neurodegenerative disease such as AD as compared to heterozygous subjects.

The term "screening" as used herein refers to a procedure used to evaluate a subject for any risk of developing a neurodegenerative disease, including the risk of an early onset of the disease. The term "age at onset" (AAO) refers to the age at which a subject is affected with a particular neurodegenerative disease. In the context of the present invention AAO and in particular an early onset of the neurodegenerative disease is understood as a risk factor for the neurodegenerative disease and can thus be screened according to the present invention. This, as onset of the disease is also crucial, as understanding the regulation of onset could make it possible to delay onset beyond an individual's normal life span. Thus, the marker or functional polymorphism of the HtrA1 gene which is determined according to the present invention may also indicate "age of onset", particularly subjects at risk for neurodegenerative diseases such as AD, with the presence of the marker indicating an earlier age of onset for the neurodegenerative disease such as AD. Of course, it is not required that the screening procedure is free of false positives or false negatives, as long as the screening procedure is useful and beneficial in determining which of those individuals within a group or population of individuals are e.g. at increased risk of AD. A screening procedure may be carried out for both prognostic and diagnostic purposes.

A prognostic method basically refers to methods used to help predict, at least in part, the course of a disease. For example, a screening procedure may be carried out on a subject that has not previously been diagnosed with a neurodegenerative disease such as AD, or does not show substantial disease symptoms, when it is desired to obtain an indication of the future likelihood that the subject will be afflicted with e.g. AD. In addition, a prognostic method may be carried out on a subject previously diagnosed with AD and/or Parkinson's disease when it is desired to gain greater insight into how the disease will progress for that particular subject (e.g., the likelihood that a particular patient will respond favourably to a particular drug treatment, or when it is desired e.g. to classify or separate AD patients into distinct and different subpopulations for the purpose of conducting a clinical trial thereon). A prognostic method may also be used to determine whether a person will respond to a particular drug. A diagnostic method refers to screening procedures carried out on a subject that has previously been determined to be at risk for a particular neurodegenerative disorder due to the presentation of symptoms or the results of another (typically different) screening test. However, the terms diagnostic method and prognostic method can also be used interchangeably as a clear delineation is sometimes difficult.

Furthermore, the inventors found that mutations respective allelic variations in regulatory elements of the HtrA 1 gene and in particular the promoter region of the HtrA1 gene constitute a particular risk factor for neurodegenerative diseases, such as in particular AD. Therefore, according to a preferred embodiment, at least one risk marker such as e.g. a mutation respective allelic variation which is located in the promoter region of the HtrA1 gene is detected in the screening method according to the present invention. It was found by the inventors that a single nucleotide polymorphism (SNP), rs11200638, in the promoter region of the HtrA1 gene constitutes an increased risk of an earlier onset of neurodegenerative diseases such as in particular AD. The wildtype has the genotype GG, wherein in rs11200638 a transition from G to A occurred (AG - heterozygous gentotype; AA homozygous genotype). This particular polymorphism rs11200638 in the promoter of the HtrA1 gene was so far only known to be linked to the eye disease AMD (Dewan et al., Science 314:989-992 (2006); Yang et al., Science 314:992-993 (2006))

The inventors proved that this single nucleotide polymorphism in the HtrA1 gene is associated with an increased risk of acquiring a neurodegenerative disease or of acquiring the disease at an earlier age and is more likely to have an increased load of amyloid ß containing neuritic plaques in the relevant regions of the brain. This SNP is in particular a marker for AD and therefore provides a suitable screening marker according to the present invention for AD. E.g. Example 1 described in further detail below identified a significant difference in the mean AAO of AD between HtrA1 GG-carriers (75.67 years) and AA/AG-carriers (72.46 years). Further studies, as shown in Examples 2-5 described in further detail below, confirmed and added further support to the identified relevance of variations in HtrA1 as a marker for AD.

Specifically, the effect on AAO of AD is more pronounced in individuals carrying the HtrA1 allele compared to individuals carrying the ApoE4 allele, which is an already established AD risk factor. In addition, levels of HtrA1-RNA as demonstrated by RealTime PCR, was significantly upregulated 2.19-fold in AD vs. control samples (see Example 6). Therefore, the body responds to the presence of APP fragments and amyloid plaques with increased synthesis of the quality control factor HtrA1. This result further indicates an additional direct link between the protein quality control factor HtrA1 and AD. Thus, HtrA1 plays an important role in the development of the AD. Also, the HtrA1-RNA levels are decreased in individuals carrying the HtrA1-promoter genotype AG (heterozygous). These results suggest that a) protein quality control is perturbed in individuals carrying the AG variation and b) decreased HtrA1 levels lead to an increased load of plaques and thus promote AD. Thus, HtrA1 in individuals carrying the HtrA1 AG allele is less able to proteolytically remove Aß peptides which then aggregate and form a larger number of plaques (Example 7). The respective promoter SNPs indicative for AD thus results in altered expression of the HtrA1 gene product and thus altered phenotype. Furthermore, a respective mutation also results in an increased neuritic plaque load in AD patients. These findings provide valuable methods of diagnosing including prognosing as well as treating AD.

Therefore, according to one embodiment, the SNP rs11200638 (on the PRSS11 locus) is detected in an individual for determining said neurodegenerative risk factor. The SNP rs11200638 indicates that a person has a higher risk of acquiring a neurodegenerative disease such as AD or of acquiring the disease at an earlier age. This risk factor also indicates that the subject carrying this mutation/allelic variation is more likely to have an increased load of neuritic plaques in the relevant regions of the brain.

According to one embodiment, the detecting step is carried out by collecting a biological sample containing nucleic acids from the subject and then determining the presence or absence of at least one risk marker such as an mutation/allelic variant in the HtrA1 gene. According to one embodiment, said allelic variant to be determined is the SNP rs11200638 as this SNP constitutes a high risk factor associated in particular with the neurodegenerative disease AD, as it in particular causes an earlier onset of the disease (see above).

There are various methods known in the state of the art that enable the analysis of a risk marker according to the present invention, which is an allelic variant polymorphism or mutation of the HtrA1 gene. These detection methods include but are not limited to a method selected from the group consisting of restriction fragment length polymorphism (RFLP) (Botstein et al, 1980), temperature gradient gel electrophoresis (DGGE) (Riesner et al, 1992), single strand conformation polymorphisms (SSCP) (Hayashi et al, 1991), heteroduplex analyses (HD) (Keen et al, 1991, Grompe, 1993), DNA sequencing (e.g. according to the Sanger method), pyrosequencing (Ronahgi et al, 1998 and 2001; Ahmadian et al, 2000), typing using molecular beacons (e.g. Tyagi, 1996 and Tyagi et al, 1998), dynamic allele specific hybridisation (DASH) (Howell et al, 1999) and amplification refractory mutation system (ARMS) (Newton et al, 1989, Sommers et al., 1989; Liu et al, 1997).

The HtrA1 genotype is preferably determined by isolating a nucleic acid from the subject and analyzing it for the particular HtrA1 risk marker of interest such as an allele polymorphism/mutation. There are various suitable methods known in the prior art that enable the analysis of an isolated nucleic acid which is preferably DNA.

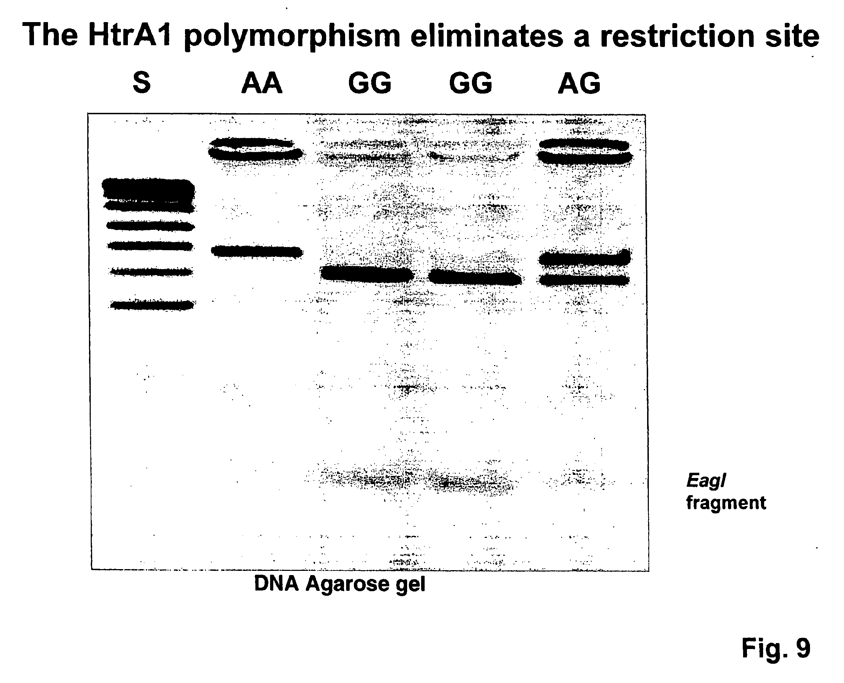

In a preferred embodiment, restriction fragment length polymorphism (RFLP) analysis is used to determine the HtrA1 genotype. This method is very convenient as it is easy to perform and thus is not error-prone. E.g., in order to determine the SNP rs11200638, a RFLP is performed using a restriction enzyme detecting a nucleic acid sequence comprising the nucleotides C**G**GCCG. The wildtype of the SNP rs11200638 is GG, wherein allelic variations constituting a risk factor for AD are AG (heterozygous) or AA (homozygous). Hence, according to this embodiment wherein the SNP rs11200638 is used as risk marker, the amplified nucleic acid is cleaved at the SNP locus in case the subject carries the wildtype and is not cleaved in this position in case the subject carries the SNP rs11200638. By this method it is also possible to differentiate whether the subject carrying the SNP rs11200638 is homo- or heterozygous. By analysing the restriction pattern, the genotype of the subject may thus be determined. Fig. 9 shows an example.

A suitable enzyme for performing a respective restriction analysis is e.g. Eagl, its isoschizomers and other enzymes recognising the corresponding sequence including BsiEl, Eael, Gdill, Sfil and Bgll and their respective isoschizomers.

Of course, it is also possible to target the allelic variation by using suitable restriction enzymes recognising and clearing the allelic sequence but not clearing the wild type sequence.

According to a preferred embodiment the screening method according to the present invention comprises the following steps:
a. Amplifying at least part of the HtrA1 gene of the subject sample using suitable oligonucleotide primers,
b. Incubating said amplified DNA with a suitable restriction enzyme for detecting the risk marker, in particular the single nucleotide polymorphism (SNP) rs11200638,
c. Determining the size of the resultant restriction fragments.

By analysing the restriction fragments it can be determined whether the subject carries the respective risk factor for a neurodegenerative disease such as AD, including the risk of AAO of said disease.

Suitable primers which can be used for determining the SNP rs11200638 are the forward primer 5'-ATGCCACCCACAACAACTTT-3' and the reverse primer 5'-CGCGTCCTTCAAACTAATGG-3'. These primers produce an amplification product of 385 nt. Restriction with e.g. Eagl results in a characteristic pattern, wherein the wildtype genotype produces fragments of 139 and 247 nt. The AG allele (heterozygous) produces three fragments of 385, 139 and 247 nt. In the AA allele (homozygous) no restriction occurs, thereby showing a 385 nt fragment only.

According to a further embodiment, the genotype of the HtrA1 gene is determined by using array systems, in particular microarrays. An array for detecting at least one risk factor associated with a neurodegenerative disease such as AD comprises a substrate carrying one or more reagents for identifying in a nucleic acid sample from a subject the occurrence of a risk factor in the HtrA1 gene that is associated with the risk of developing or expediting the neurodegenerative disease such as AD. The risk factor can be e.g. an allelic variation/mutation. Said array preferably comprises reagents such as oligonucleotides detecting the single nucleotide polymorphism (SNP) rs11200638 of the HtrA1 gene.

Several methods are known to perform a SNP typing by using a support, which is carrying e.g. SNP specific oligonucleotides which hybridise to the target nucleic acid (e.g. by Affymetrix). The sensitive and high-throughput nature of hybridization-based DNA microarray technology provides an ideal platform for such a screening application as it allows interrogating up to hundreds of thousands of single nucleotide polyphorphisms (SNPs) in a single assay. Hence, several risk factors for neurodegenerative diseases such as AD may be determined in one expression system. For example, multiple sets of short oligonucleotide probes for each known SNP marker can be used in order to securely determine the HtrA1 genotype of the subject. Further details of array based SNP analysis methods are e.g. disclosed in Hacia, 1999; Pease et al, 1994; Sosnowski et al, 1997; Gilles et al, 1999 and Ngyen et al, 1999.

These allelic specific hybridisation reactions may also be combined with enzymatic methods. Suitable methods are disclosed e.g. in Landegren et al, 1988; Wu et al, 1989; Parik et al, 1993; Samiotaki et al, 1994; Grossman et al, 1994; Day et al, 1995; Lou et al, 1996 and Gunderson et al, 1998. Further SNP detection methods involving the use of chips are disclosed in Syvänen et al 1990, Pastinen et al, 1997, Pastinen et al 2000 and Dubiley et al, 1999.

It was also predicted by the inventors that the HtrA1 expression is reduced in the AG or AA genotype of rs11200638. Therefore, an alternative screening method which can also be used in addition to the above described screening method is to measure the HtrA1 expression. This can be done e.g. by determining the protein concentration in a sample e.g. via ELISA or other methods such as quantitative Western blotting in order to detect the presence or absence of the HtrA1 risk factor for a neurodegenerative disease. Detection of HtrA 1 expression may also be performed by suitable methods for detecting mRNA levels in a sample. E.g. a real-time polymerase chain reaction may be combined with a reverse transcription polymerase chain reaction to quantify messenger RNA (mRNA), enabling the quantification of relative gene expression.

As the development of neurodegenerative diseases such as AD and Parkinson's disease and the AAO of such diseases is influenced by many different risk factors, it is preferred according to the present invention that the presence or absence of at least one or more additional markers linked to said neurodegenerative disease(s) is or are detected in the screening. Hence, the occurrence of a further risk factor such as an allelic variant also associated with the risk of developing or expediting the neurodegenerative disease(s) under analysis (in particular AD) besides the HtrA1 gene is analysed. Suitable respective additional markers constitute e.g. risk markers of the ApoE gene.

Diagnostic kits may be used for performing the screening method described above. A respective diagnostic kit is useful for screening risk factors of neurodegenerative diseases such as AD and in particular for determining the risk of suffering from AAO of the respective disease.

Kits for determining if a subject is or was (in the case of deceased subjects) afflicted with or is or was at increased risk of developing a neurodegenerative disease such as AD will include at least one reagent specific for detecting for the presence or absence of at least one risk marker as described herein and instructions for observing that the subject is or was afflicted with or is or was at increased risk of developing the neurodegenerative disease such as AD if at least one respective risk marker is detected. The kit may optionally include one or more nucleic acid probes for the amplification and/or detection of the risk marker by any suitable techniques (in particular the ones described above), with PCR being currently preferred.

According to one embodiment, the diagnostic kit allows the detection of a SNP in the promoter region of the HtrA1 gene, in particular of SNP rs11200638. Preferably, the three HtrA1 genotypes GG, AG, and AA are detected with the kit. In case the genotype is to be determined by an enzymatic method, the test may be based on determining the presence or absence of one cleavage site for the restriction enzyme *Eagl* or any other restriction enzyme that detects a C**G**GCCG nucleotide sequence (for suitable examples please also see above). Thereby, the marked HtrA1 **G** allele can be identified. As described above, it is also possible to detect the allelic variant by using appropriate restriction enzymes. Further details and suitable primers are described above and can be used in a respective kit. The kit may also include suitable enzymes for amplifying and restricting nucleic acids.

Detections by RFLP can be replaced by a large number of methods, including high throughput methods available for detection of SNPs and/or other allelic variations, for example and without limitation by the methods described above and described in the Examples below.

In one embodiment, DNA from a sample is sequenced (resequenced) by any method to identify a SNP or small allelic variation. A large variety of resequencing methods are known in the art, including high-throughput methods. Amplification-based methods also are available to identify allelic variations, such as SNPs, include, without limitation: PCR, reverse transcriptase PCR (RT-PCR), isothermic amplifications, nucleic acid sequence based amplification (NASBA), 5' fluorescence nuclease assay (for example TAQMAN assays), molecular beacon assays and rolling circle amplifications. Other methods, such as Restriction Fragment Length Polymorphisms (RFLP), also maybe employed as they are appropriate and effective to identify variant allele(s). Assays may be multiplexed, meaning two or more reactions can be carried out simultaneously in the same physical location, such as in the same tube or position on an array, as long as the reaction products of the multiplexed reactions can be distinguished.

As a non-limiting example, TAQMAN or molecular beacon assays can be multiplexed by use of and by monitoring of accumulation or depletion of two different fluorochromes corresponding to two different sequence-specific probes. Hence, suitable molecular beacons allowing to identify the genotype of the subject (in particular in the locus of rs11200638) can be provided in the kit. In most cases, the appropriate method is dictated by personal choice and experience, equipment and reagents on hand, the need for high throughput and/or multiplexed methods, cost, accuracy of the method, and the skill level of technicians running the assay. Design and implementation of those techniques are known and are well within the abilities of those of average skill in the art.

The kit can be used for example for identifying a human subject having an increased risk of developing a neurodegenerative disease such as AD or of acquiring said disease at an earlier age and is more likely to have an increased load of Aß containing neuritic plaques in the relevant regions of the brain. The methods comprise identifying a nucleic acid sample from the subject the occurrence of a risk marker such as e.g. an allelic variant or a specific haplotype (comprised of several allelic variants) of HtrA1. A specific single nucleotide polymorphism that was identified as a high risk factor is rs11200638 (see above) which is therefore detected according to a preferred embodiment of the invention.

Said kit may comprise at least one of the following components:
a) primers for amplifying at least part of the HtrA 1 gene, in particular primers for amplifying at least part of the promoter region of the HtrA1 gene encompassing the site of the single nucleotide polymorphism (SNP) rs11200638;
b) at least one enzyme for nucleic acid amplification;
c) at least one enzyme suitable for performing a RFLP analysis for identifying at least one risk marker;
d) instructions for use.

It was also demonstrated by the inventors that an increased load of neuritic plaques and increase of neurofibrillary tangles is found in rs11200638 carriers. Immunophenotypes of AD brains were analyzed quantitatively by determining the number and tissue area covered by cortical Aß4-immunoreactive plaques and Aß4/tau-positive neuritic plaques, as well as the number of neurofibrillary tangles (see Example 7). The number of neuritic plaques as defined by Aß-positive plaques containing tau-positive profiles is 3-fold higher in the frontal cortex of individuals carrying the HtrA1-promoter genotype AG compared to the GG genotype. This difference was also statistically significant in the temporal cortex. The number of tau-positive neurofibrillary tangles in the frontal cortex of AD patients with the HtrA1 AG genotype is also significantly increased compared to HtrA1 GG-carriers (p<0.01). This difference was also statistical significant in the temporal cortex.

The identification of a SNP in the promoter region of the HtrA1 gene as the cause of earlier onset and increased neuritic plaque load in patients also provides novel therapeutic approaches for treating respective diseases. The specific advantage of this invention is that a genetic disposition such as the SNP rs11200638 is directly correlated with the risk or earlier onset of the neurodegenerative disease. This SNP is also directly correlated to physical changes, such as a change in the level of plaque formations that are the hallmark of e.g. AD. These important findings open up new opportunities for therapy.

A further important finding of the present invention is that the long sought tau protease that is capable of digesting tau, tau aggregates and hyperphosphorylated tau and aggregates of hyperphosphorylated tau is the serine protease HtrA1. The inventors identified the serine protease HtrA1 as the protease that digests tau as well as hyperphosphorylated or pseudohyperphosphorylated tau. As the dissociation of phosphorylated tau from microtubules and its subsequent aggregation leads to tangle formation in the cytosol of neurons is one hallmark of Alzheimer's disease and other tauopathies, the degradation of tau by HtrA1 directly implicates novel approaches for therapeutic intervention. Examples 11 and 15 (see below) revealed that the entire region of tau responsible for aggregation and formation of PHFs and tangles is completely degraded by HtrA1 into small fragments that are accordingly unlikely to result in aggregation and thus in neuronal cell death. The repeats responsible for aggregation are shown below in bold face and are underlined; the sequence responsible for self assembly of tau is given in bold face. Arrows indicate identified cleavage sites of HtrA1. The products of the digests of pseudohyperphosphorylated tau are shown.

self
repeat assembly repeat
↓ ↓ ↓ ↓↓↓↓ ↓ ↓ ↓ ↓ ↓ ↓ ↓ ↓
VKSKIGSTENLKHQPGGGKVQIVYKPVDLSKVTSKCGSLGNIHHKPGGGQVEVKSE
↓ ↓ ↓ ↓ ↓↓↓
KLDFKDRVQSKIGSLDNITHVPGGG
repeat

Further studies (see examples 12 and 13) using chemically cross-linked aggregates of tau and of pseudohyperphosphorylated tau showed that these tau isoforms are degraded in a similar manner and with similar efficiencies. These data confirm and add further support to the identified relevance of the role of HtrA1 as a disease modifying factor in AD and related diseases. In particular, these studies show that the upregulation of HtrA1 will be beneficial to cells already containing significant amounts of tau aggregates and thus for patients already suffering from the disease. An additional line of evidence (see example 14) indicates the physiological significance of these data. It is shown that cells overexpressing pseudohyperphosphorylated tau respond with the upregulation of the protein quality control protease HtrA1 in an attempt to clear the toxic tau isoform that is leading to cell death if clearance is unsuccessful. These data demonstrate that HtrA1 is an important therapeutic target.

Thus, also therapeutic strategies for curing AD and related diseases (such as tauopathies) derived from these findings are described. The specific advantage of this invention is that tau degradation is a disease modifying event that is directly correlated with earlier onset of the disease in combination with physical changes that is the level of intracellular tangle and extracellular plaque formation that are the established hallmarks of the disease. In addition, the fact that even chemically cross-linked tau aggregates can be degraded points to the possibility of curing patients already suffering from AD. The novel approach for therapeutic intervention that focuses on the proteolytic clearance of aggregated tau (present as intracellular tangles or extracellular aggregates) is based on activation of HtrA1 on either the transcriptional or the enzymatic levels. Transcriptional activation can be mediated for example by overexpressing or modulating a transcriptional activator of the HtrA1 promoter. Other avenues include enzymatic upregulation of HtrA1 that can be accomplished by using activating compounds that bind to the PDZ domains of HtrA1 or other regions of the protein that are involved in the regulation of its protease activity. Such compounds can be peptides or derivatives of peptides, natural compounds and their derivatives as well as chemical compounds.

Hence, a novel approach for therapeutic intervention is based on activation of HtrA1 e.g. on either the transcriptional or enzymatic level. Transcriptional activation can be brought about for example by overexpressing or modulating a transcriptional activator or transcriptional activators of the HtrA1 gene that are binding less efficiently to the HtrA1 promoter carrying the AG or AA genotype. These transcriptional activators can be e.g. upregulated by stimulation of their own promoters. Upregulation can also occur on the post transcriptional levels for example by increasing their stability, their half-life or the level of phosphorylation or other post translational modifications, stimulating their biological activity.

Enzymatic upregulation of HtrA1 in order to counteract e.g. the down-regulating effect of SNP rs11200638 can also be accomplished by e.g. using HtrA1 activating compounds that bind to the PDZ domains of HtrA1 or other regions of the protein that are involved in the regulation of protease activity. Such compounds can be peptides or derivatives of peptides, natural compounds and their derivatives as well as chemical compounds such as small molecules. Respective compounds can be identified by using established screening methods.

The frequency of occurrence of the wt GG allele increases from 58.7% in the population of the <50 year olds to 72% of the >71 year olds and the AG or AA alleles decrease from 41.3% in the <50 year olds to 28% of the >71 year olds. This result indicates that the life span of individuals carrying the AG or AA alleles in the HtrA1 promoter is decreased. Since the life span of individuals carrying the AG or AA alleles in the HtrA1 promoter (SNP rs11200638) is decreased, the above mentioned therapeutic compounds can also serve as life extending drugs for non-AD sufferers carrying this or other alleles that reduce HtrA1 levels or function.

Accordingly, also methods for therapeutic intervention focusing on the proteolytic elimination of tau are provided with the present invention for patients at risk of developing AD or for patients already suffering from AD. These methods comprise activation of HtrA1 on the enzymatic or the genetic levels.

Also the identification of the HtrA1 protein as the tau protease provides novel approaches for therapeutic intervention that focus on the proteolytic clearance of tau that is not associated with microtubules but is aggregated in the cytosol (tangles) or present as extracellular aggregates. This therapy is according to the teachings of the present invention also based on an activation of HtrA1 on the transcriptional and/or the enzymatic levels. As is outlined above, transcriptional activation can be brought about for example by overexpressing or modulating a transcriptional activator or transcriptional activators that are binding to the HtrA1 promoter. These transcriptional activators can be either upregulated by stimulating their own promoters or on the post transcriptional levels for example by increasing their stability that is half life or the level of phosphorylation or other post translational modifications that stimulate their biological activity. Approaches for enhancing HtrA1 expression may also include the manipulation of chromatin composition at the HtrA promoter allowing transcription factors to better access their target. Enzymatic upregulation of HtrA1 can also be accomplished by using activating compounds that bind to the PDZ domains of HtrA1 or other regions of the protein that are involved in the regulation of protease activity. Such compounds can be peptides or derivatives of peptides, natural compounds and their derivatives as well as chemical compounds.

The teachings provided with the present invention also provide the possibility to treat diseases that are based on protein folding defects leading to protein or protein fragment aggregation or diseases in which protein aggregates are a major side effect by activating ATP independent protein quality control factors such as HtrA1. These protein folding or amyloid diseases include but are not limited to Prion disease such as Creutzfeldt Jacob; Huntington's disease, tauopathies, and alpha-Synucleinopathies such as Parkinson's disease (also see above). Diseases in which protein aggregates are a major side effect are endocrine and other cancers that are known to progress more aggressively and have an increased metastasis when they lead to protein aggregate formation.

Accordingly, the present invention also pertains to the use of a compound enhancing HtrA1 enzyme activity, for the manufacture of a medicament for the treatment or prevention of a tauopathy, in particular Alzheimer's disease.

Furthermore, the present invention also pertains to the use of a compound enhancing HtrA1 enzyme activity, for the manufacture of a medicament for proteolysing or degrading tau, phosphorylated tau, hyperphosphorylated tau, and/or tau aggregates, phosphorylated tau aggregates and/or hyperphosphorylated tau aggregates.

According to one embodiment, said compound activates or stabilises the enzyme HtrA1. Thus the term "enhancing HtrA1 enzyme activity" basically refers to an increase in HtrA1 activity in a sample or cell. Furthermore, several measures may be used such as enhancing gene expression, inhibiting HtrA1 degradation or HtrA1 mRNA degradation or direct activation of the HtrA1 enzyme by an activator.

Neurodegenerative disease can be amyloid diseases, in particular selected from the group consisting of Prion diseases such as Creutzfeldt Jacob disease; Huntington's disease; Tauopathies; Alpha-Synucleinopathies, in particular Parkinson's disease and Lewy body dementia and Alzheimer's disease. The present invention is directed to tauopathies as neurodegenerative diseases.

According to the invention, an activator of HtrA1 is used, which comprises or consists of a sequence selected from the group consisting of:
a) DQLAFHQFYI
b) XXXDSRIWWV, wherein X is a natural or unnatural amino acid
c) KKKDSRIWWV.

The invention also pertains to the in vitro use of HtrA1 or a functional variant thereof having HtrA1 activity for proteolysing or degrading tau, phosphorylated tau, hyperphosphorylated tau, and/or tau aggregates, phosphorylated tau aggregates and/or hyperphosphorylated tau aggregates.

A pharmaceutical composition may comprise an HtrA1 activator as defined above. The invention furthermore pertains to an HtrA1 activator as described above for treating or preventing a tauopathy, preferably Alzheimer's disease. A HtrA1 activator as described herein is particularly useful for proteolysing or degrading tau, phosphorylated tau, hyperphosphorylated tau, and/or tau aggregates, phosphorylated tau aggregates and/or hyperphosphorylated tau aggregates. This usually when present in a sample/environment where HtrA1 is present, such as an *in vitro* or *in vivo* experiment or during therapeutic applications.

The invention also pertains to the use of HtrA1 in a screening assay for identifying a compound capable of inhibiting or promoting proteolysis or degradation of tau, phosphorylated tau, hyperphosphorylated tau, and/or tau aggregates, phosphorylated tau aggregates and/or hyperphosphorylated tau aggregates. A respective screening assay/setting e.g. would comprise HtrA1, a potential activator or inhibitor of HtrA1 and a reagent selected from the group consisting of tau, phosphorylated tau, hyperphosphorylated tau, pseudohyperphosphorylated tau and/or tau aggregates, phosphorylated tau aggregates, hyperphosphorylated tau aggregates and/or pseudohyperphosphorylated tau aggregates.

The following figures depict certain embodiments and findings of the invention.

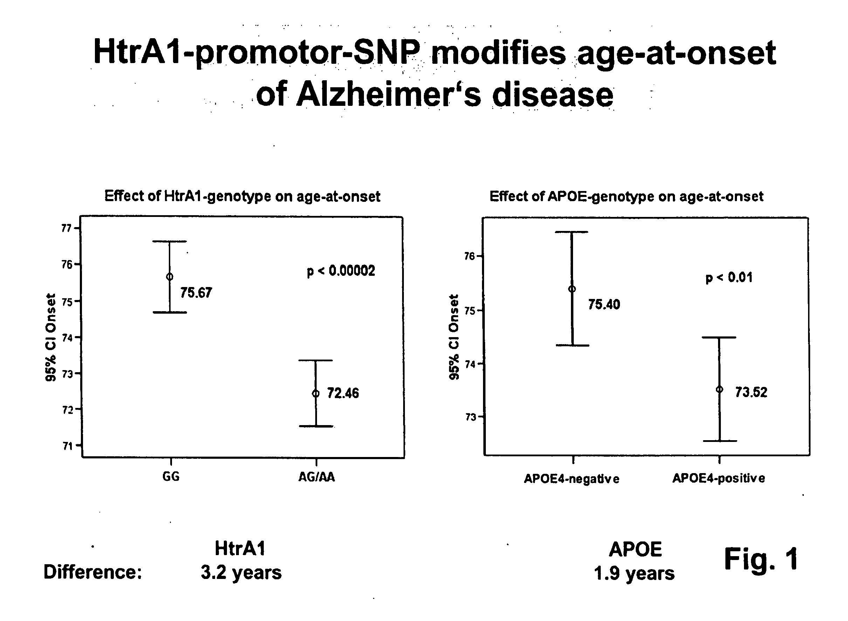

**Fig.1** shows that the HtrA1 promoter SNP rs11200638 is associated with AAO of AD. In a search for a genetic basis for the involvement of the serine protease HtrA1 in AD we detected that the HtrA1 promoter SNP rs11200638 modifies AAO in patients. In our exploratory dataset of 203 AD patients with AAO 65 to 86 years there was a significant difference (p<0.00002) in mean AAO between HtrA1 GG-carriers (75.67 years) and AA/AG-carriers (72.46 years). A dose-dependent effect on AAO of the A-allele was absent, however the number of AA-carriers was low (n=7). Comparing the effects of the HtrA1 SNP with the effects of the well established ApoE4 allele causing a 1.9 years earlier onset indicates that the effects of the HtrA1 SNP are stronger compared to ApoE4.

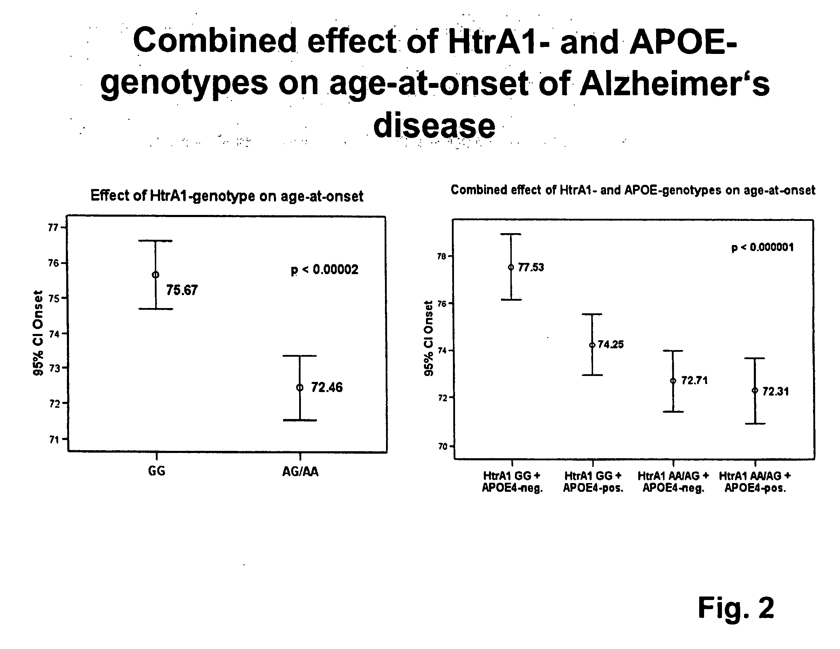

**Fig. 2** shows that the HtrA1 promoter SNP rs11200638 and APOE4 have additive effects on AAO of AD. When combining the effects of HtrA1- and APOE-genotypes on AAO, we detect for individuals that are genetically wild type in both loci an AAO of 77.53 years, for HtrA1 wt (GG) ApoE4 of 74.25 years, for HtrA1 AG ApoE wt of 72.71 and for HtrA1 AG ApoE4 of 72.31 years (p<0.000001).

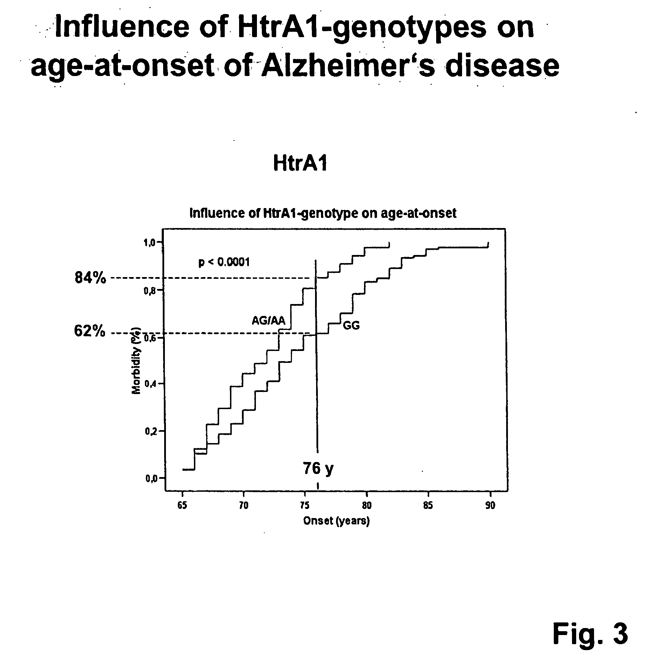

**Fig. 3** shows the influence of HtrA1-genotype on AAO. AAO for subjects with the GG (blue curve) or AG/AA (green curve) genotypes are shown. Onset curves were estimated by Kaplan-Meier product limit distributions. For example, at age 76, an estimated 62% of subjects with HtrA1 GG genotype were diagnosed with AD compared to 84% of subjects without HtrA1 GG genotype.

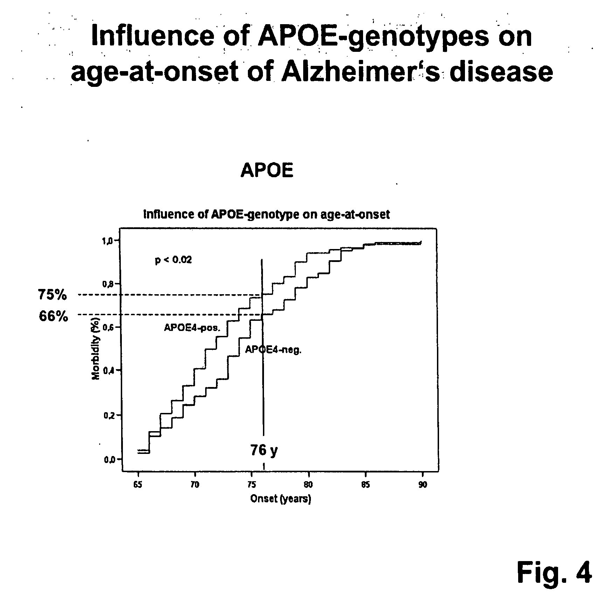

**Fig. 4** shows the influence of APOE-genotype on AAO. Compared to the HtrA1 effect with a difference of 22% between diseased subjects with GG versus AG/AA genotype at 76 years of age (Fig. 3), the APOE effect on AAO is less prominent with a 9% difference between diseased subjects without APOE4 versus with APOE4 at age 76.

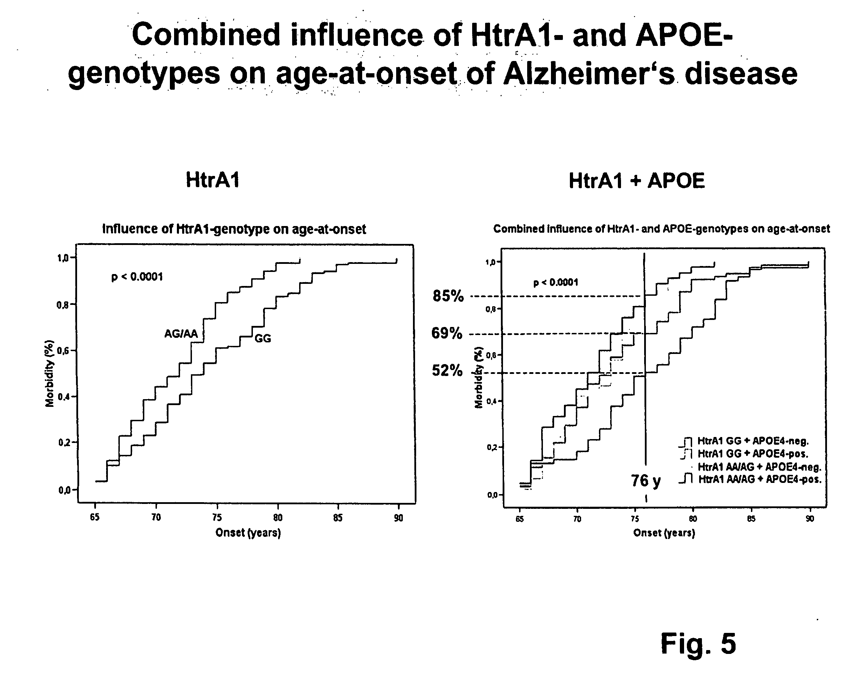

**Fig. 5** shows the combined influence of HtrA1- and APOE-genotypes on AAO. Onset curves were estimated by Kaplan-Meier product limit distributions. For example, at age 76, an estimated 52% of subjects with a combination of HtrA1-GG/APOE4-negative were diagnosed with AD compared to 69% of HtrA1GG/ APOE4-positive individuals and 85% of subjects that carry the HtrA1AG/AA genotype regardless of the APOE genotype. This further strengthens, that the HtrA1 effect on AAO is even more pronounced compared to the APOE effect.

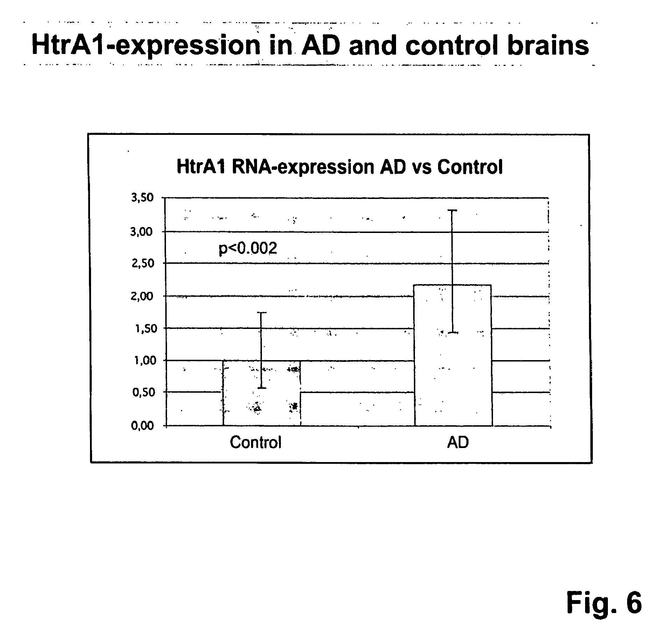

**Fig. 6** shows the HtrA1-expression in AD and control brains. Levels of HtrA1-RNA as revealed by RealTime PCR was significantly upregulated 2.19-fold in AD vs. control samples (p<0.002).

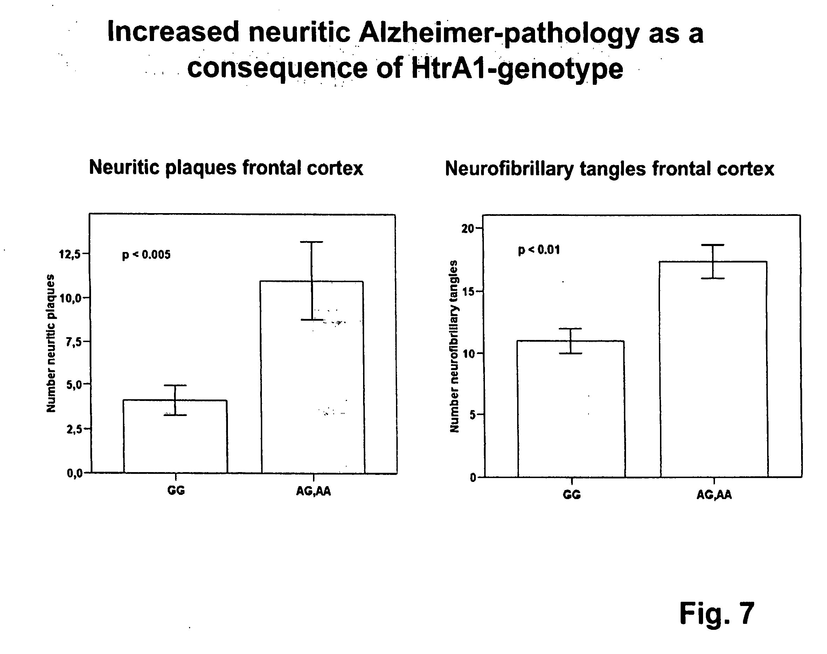

**Fig. 7** shows that increased neuritic AD-pathology is a consequence of HtrA1-genotype. Fig 7 left hand side shows that the number of neuritic plaques as defined by Aß-positive plaques containing tau-positive profiles is 3-fold higher in the frontal cortex of individuals carrying the HtrA1-promoter genotype AG compared to the GG genotype (p<0.005). This difference was also statistically significant in the temporal cortex (data not shown). Fig 7 right hand sight shows that the number of tau-positive neurofibrillary tangles in the frontal cortex of AD patients with the HtrA1 AG genotype is significantly increased compared to HtrA1 GG-carriers (p<0.01). This difference was also statistical significant in the temporal cortex (data not shown).

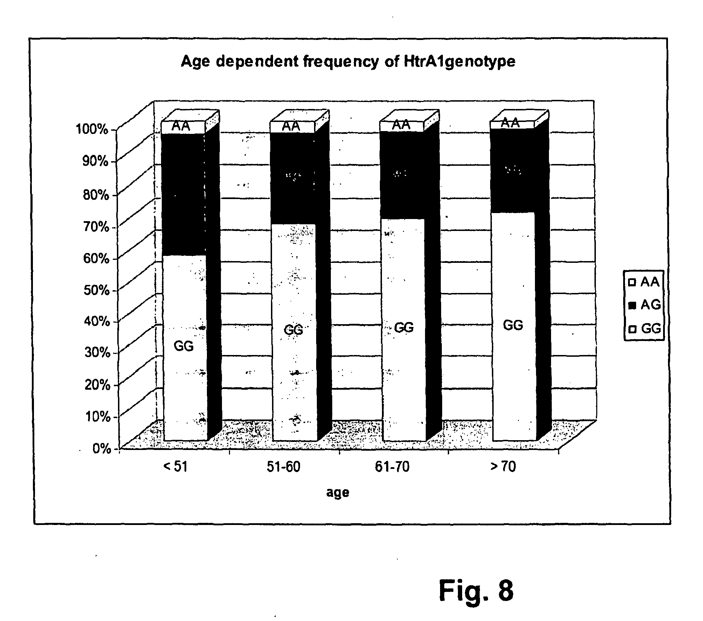

Fig. 8 and the subsequent table 1 show that the life span is decreased in AG and AA carriers compared to wildtype GG carriers.

**Table 1:**

| | up to 50 | | | 51 to 60 | | | 61 to 70 | | | over 71 | |
|---|---|---|---|---|---|---|---|---|---|---|---|
| Genotype | n | % | | n | % | | n | % | | n | % |
| **GG** | 44 | **58,7** | | 39 | **68,4** | | 44 | **69,8** | | 59 | **72,0** |
| **AG** | 28 | **37,3** | | 16 | **28,1** | | 17 | **27,0** | | 21 | **25,6** |
| **AA** | 3 | **4,0** | | 2 | **3,5** | | 2 | **3,2** | | 2 | **2,4** |

The frequency of occurrence of the wt GG allele increases with age of the individuals by 13% while frequency of occurrence of the AG or AA alleles decreases by 13%. This result indicates that the life span of individuals carrying the AG or AA alleles in the HtrA1 promoter is decreased.
**Fig.9****.** shows the result of a restriction analysis according to the invention. The relevant part of the HtrA1 promoter was amplified by PCR (see above, 385nt). The purified PCR fragment was digested with Eagl and loaded on an agarose gel. DNA staining was done with ethidium bromide. The restriction pattern for the GG, AG and AA genotype is shown. As can be seen, the genotype can be determined based on the restriction pattern.

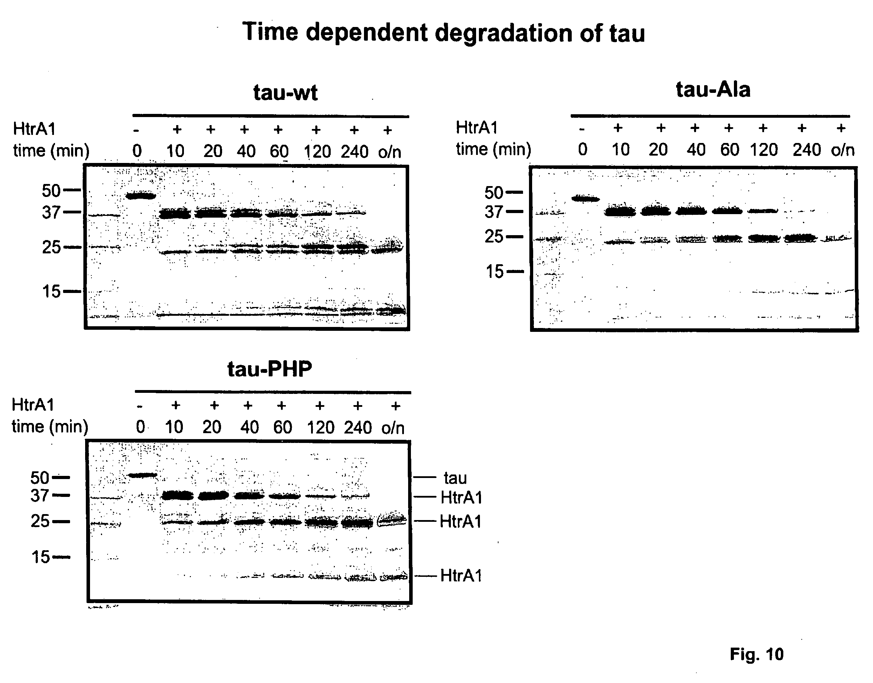

**Fig 10****:** shows the time dependent degradation of tau by HtrA1. Purified recombinant tau protein was incubated with purified recombinant HtrA1 for 0, 10, 20, 40, 60, 120, 240 minutes and overnight at 37°C. Samples were loaded on a 12% SDS-PAGE. As control HtrA1 and tau were loaded separately on the gel. The gel was stained with Coomassie Blue. HtrA1 autodigests itself, producing HtrA1 fragments migrating at 25 kDa and less than 10 kDa (indicated in the tau-PHP digests). Tau-wt and tau-Ala degradation showed no specific cleavage products. In contrast, tau-PHP degradation yielded two bands migrating between the 15 and 25 kDa markers.

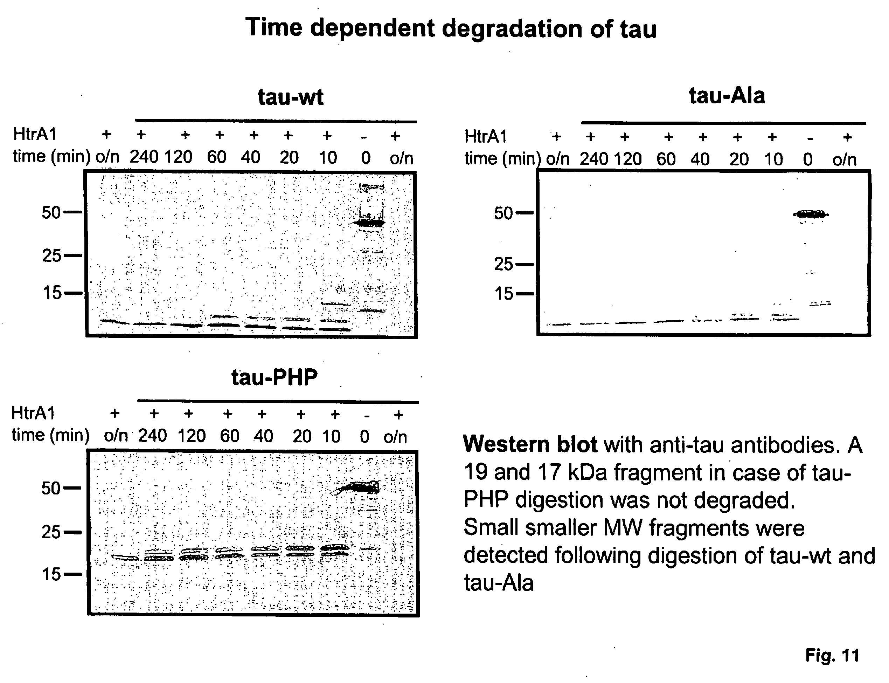

**Fig 11****:** shows the time dependent degradation of tau (Western blots). Immunoblotting analysis of proteolysis reactions described in example 10. Samples were loaded on a 12% SDS-PAGE and blotted on a nitrocellulose filter. A polyclonal anti-tau antibody was used for the detection of tau protein and degradation products. Tau-wt and tau-Ala degradation showed small degradation bands migrating near or below the 15 kDa marker. In case of tau-PHP degradation products migrated between the 15 and 25 kDa marker. These bands correspond with the N-terminal regions of tau for which no cleavage products could be identified (Examples 11-14). As PHP tau contains additional 10 negatively charged residues, it might be that the small tau fragments detected are in fact identical although migrating differently on SDS PAGE.

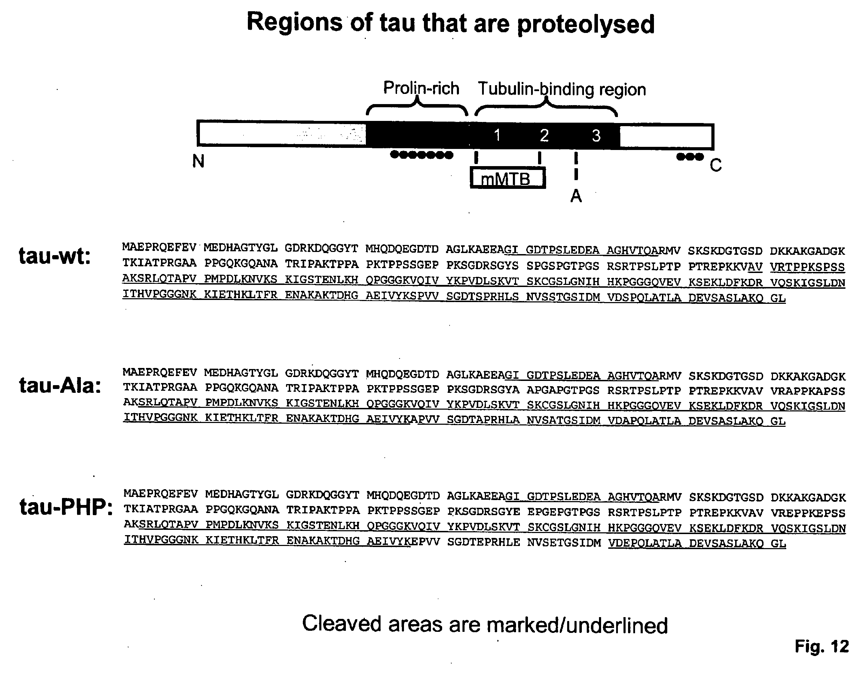

**Fig. 12****:** shows regions of tau that are proteolysed. Mass Spectrometry results of digested tau protein by HtrA1. The three tau proteins (100 µg) were incubated with HtrA1 (10 µg) overnight at 37°C. Subsequently, samples were precipitated with acetone and the supernatant containing the proteolytic products was analysed by MALDI-TOF. Large fragments and fragments smaller than 5 amino acids were not detected in MALDI-TOF. Regions of tau that were cleaved by HtrA1 are underlined.

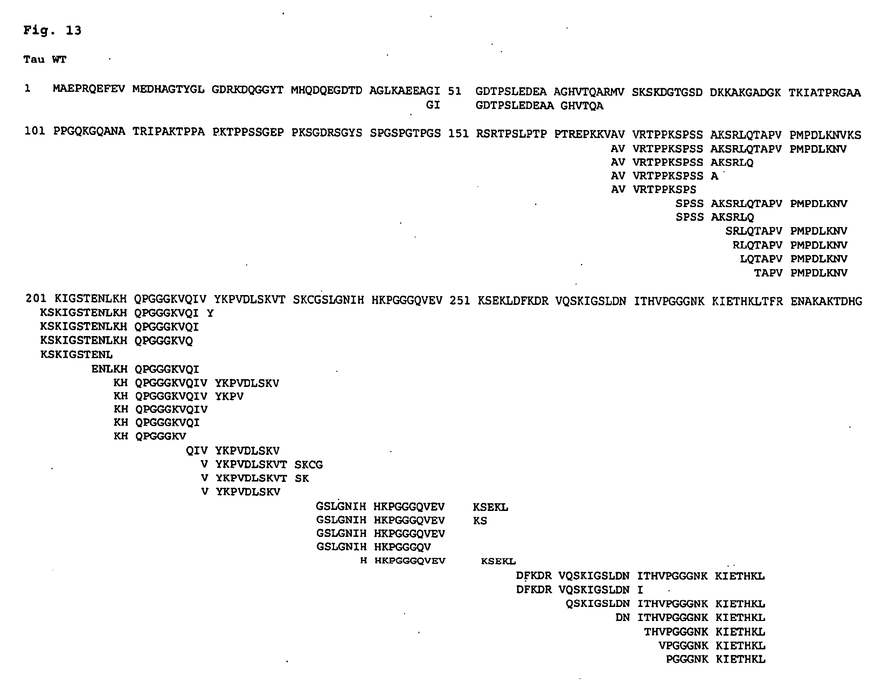

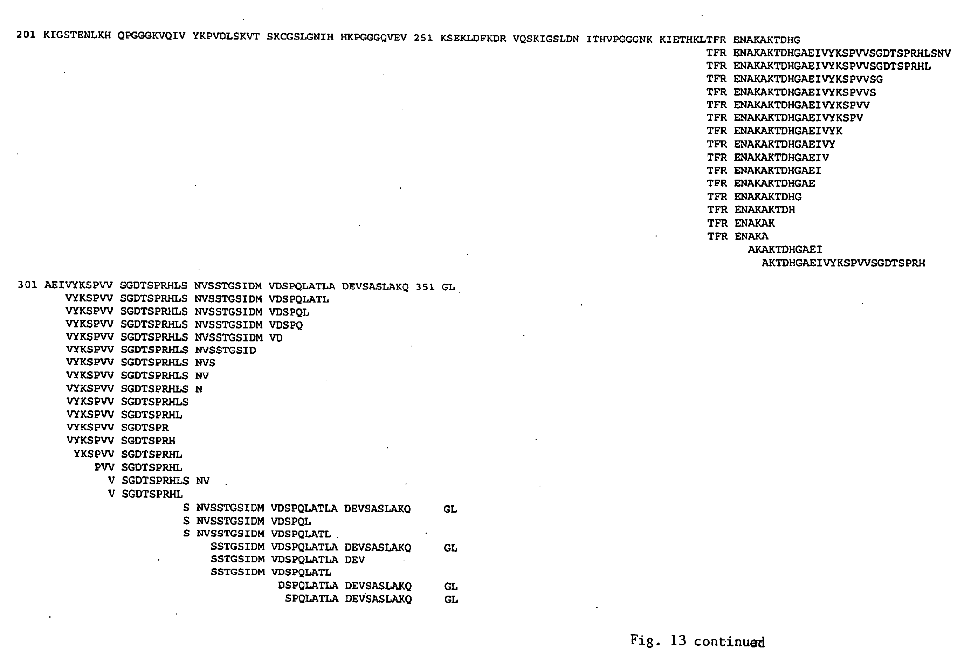

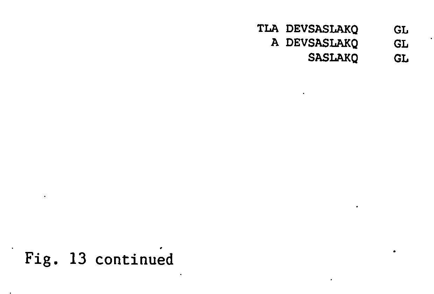

**Fig. 13****:** shows the identified cleavage products of Tau-WT. Purified wildtype tau was digested with HtrA1 and cleavage products were identified as described in Example 11. The individual tau fragments identified are shown below the sequence of the full length substrate.

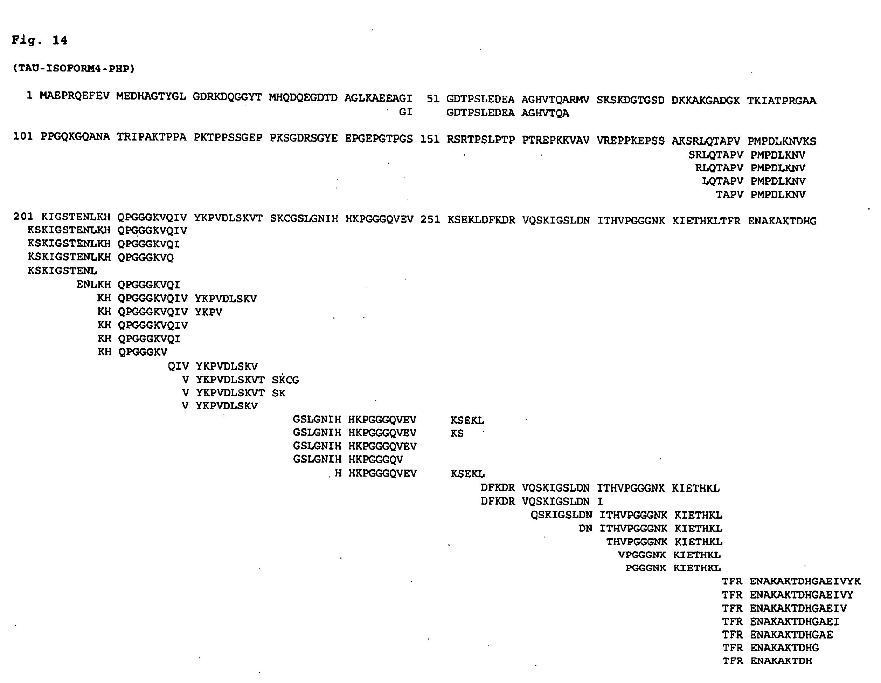

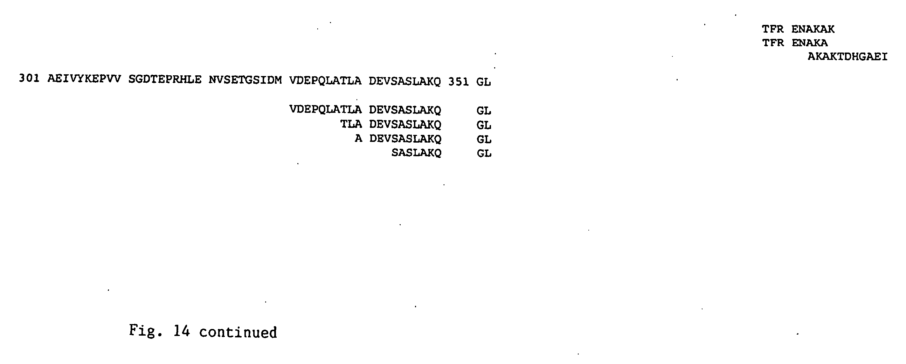

**Fig 14****:** shows the identified cleavage products of Tau-PHP. Purified pseudohyperphosphorylated tau was digested with HtrA1 and cleavage products were identified as described in Example 11. The individual tau fragments identified are shown below the sequence of the full length substrate.

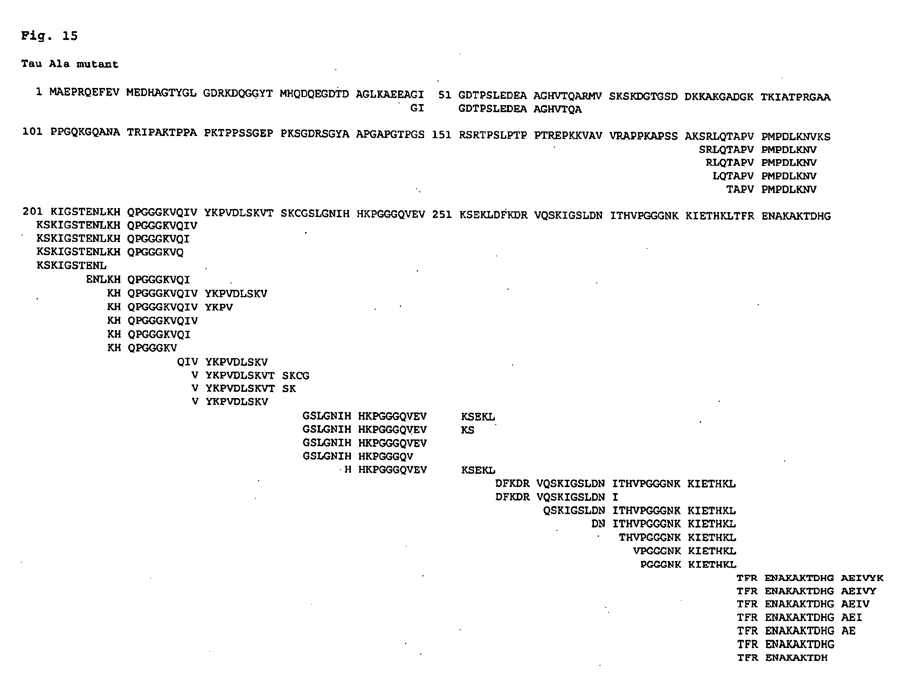

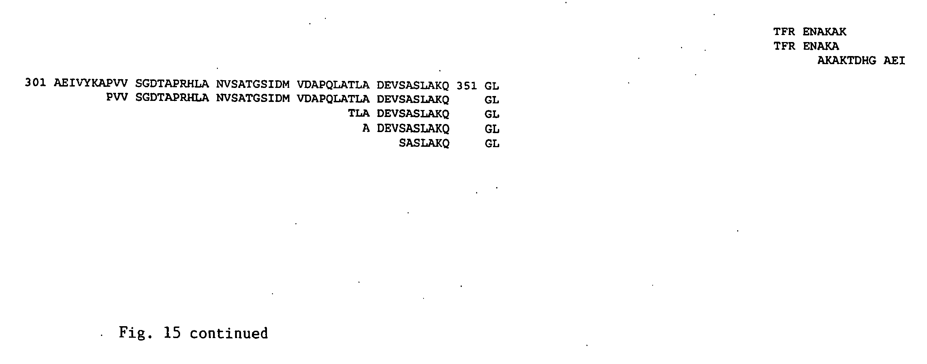

**Fig 15****:** shows identified cleavage products of Tau-Ala. Purified tau containing Ala substitutions instead of Glu substitutions introduced in PHP tau was digested with HtrA1 and cleavage products were identified as described in Example 11. The individual tau fragments identified are shown below the sequence of the full length substrate.

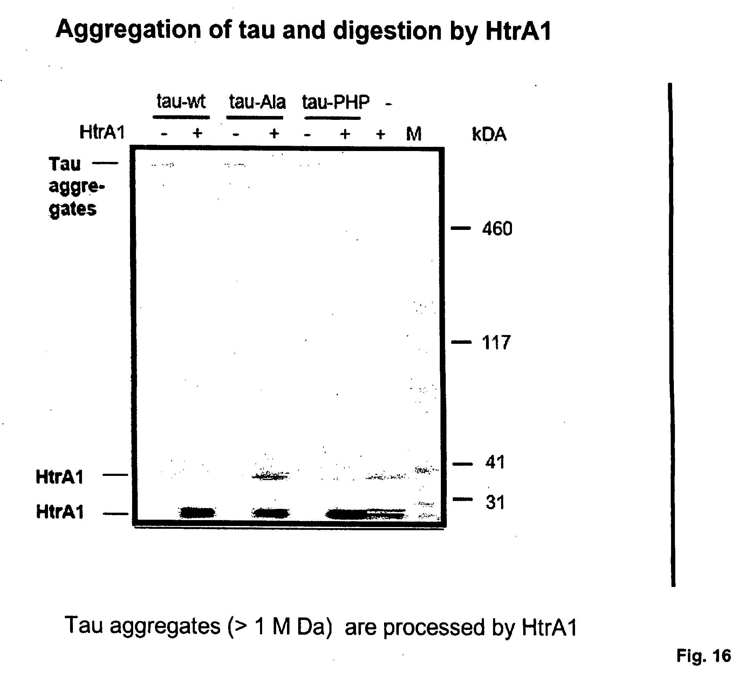

**Fig 16****:** shows aggregation of tau and digestion by HtrA1. Purified tau aggregates. Aggregation was detected by incubating Tau in phosphate buffer (50 mM, pH 7.5) containing 1% formaldehyde overnight at 37°C. Aggregated tau migrates on the very top of the gel indicating that the aggregates are > 1 mDa.
Degradation of aggregated tau by HtrA1. Tau was precipitated with TCA dissolved in Tris HCl buffer (50 mM, pH 8) and incubated with HtrA1 overnight at 37°C. As control HtrA1 and tau were incubated separately. Samples were loaded on a Tris-Tricine gel. All three tau proteins aggregated and were efficiently processed by HtrA1. No tau degradation band was detectable. HtrA1 and its degradation band were detected at 37 kDa at 25 kDa, respectively.

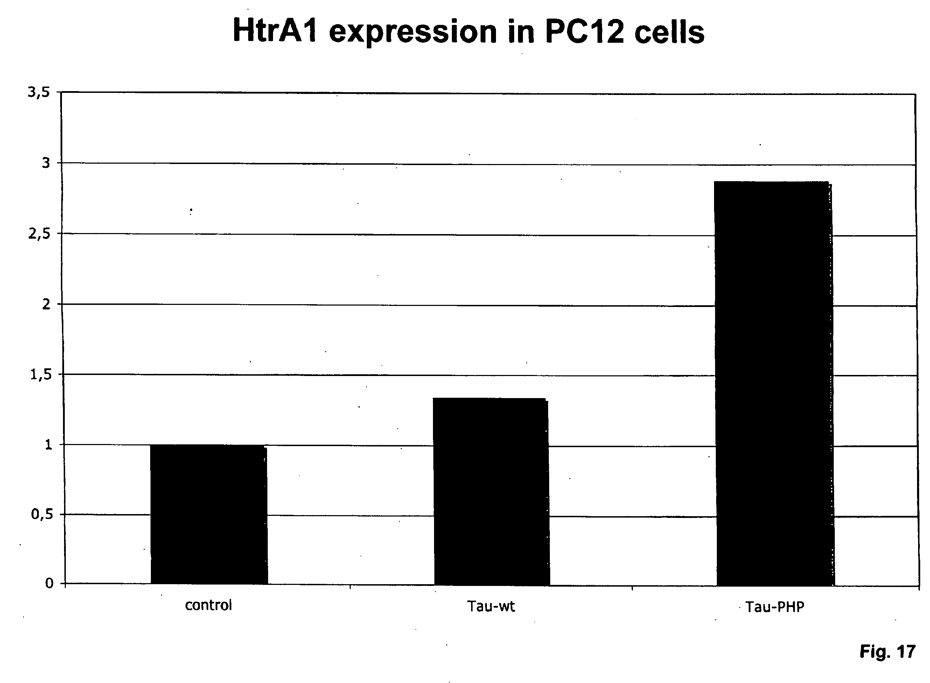

**Fig 17****:** shows the HtrA1 expression in PC12 cells. Analysis of HtrA1 expression in PC12 (rat neuronal) cells overexpressing the tau-wt protein, the tau-PHP protein or containing the empty vector. The HtrA1-RNA level was analysed by quantitative RT-PCR. HtrA1 level was significantly upregulated (2.9-fold) in PC12 cells overexpressing the tau-PHP protein compared to the empty vector control. Overexpression of tau-wt in PC12 cells led only to a slight increase 1.3-fold of HtrA1-RNA compared to the empty vector control.

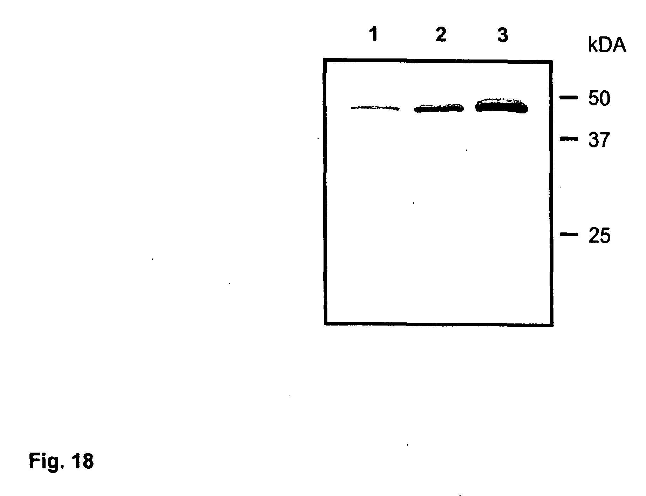

**Fig. 18****:** shows HtrA1 activation by peptides. Purified wt-tau (2 µg) was incubated with HtrA1 (0.04 µg) for 10 minutes at 37°C. HtrA1 was preincubated with different peptides for 10 minutes at 37°C. All peptides were dissolved in DMSO and added to a final concentration of 200 µM. Wt-tau was incubated with DQLAFHQFYI (lane 1), KKKDSRIWWV (lane 2) and with HtrA1 and DMSO as a control (lane 3). Samples were loaded on a SDS/PAGE. The gel was stained with Coomassie Blue.

### EXAMPLES

### Example 1

### Subjects for SNP Genotyping

The study included a total of 222 patients with AD, which were recruited at Munich. Most patients were of European ancestry. Diagnosis of AD was established according to the NINCDS-ADRDA criteria (McKhann et al., Neurology 34, 939-944 (1984)) at all participating sides. Information on AAO of the disease was obtained from an informant. AAO was defined by the appearance of first clinical symptoms. The control group, which included 227 individuals, were matched for geographical location and ethnicity and consisted of cognitively healthy, age matched subjects, who were recruited from the memory clinic and community based geriatric day-care unit.

### Example 2

### SNP Genotyping

DNA was obtained from peripheral blood lymphocytes or brain tissue samples according to standard procedures. Genotyping of the HtrA1 SNP rs11200638 was performed by RFLP analysis (Yang et al., Science 314, 992-993 (2006)). Oligonucleotide primers, forward 5'-ATGCCACCCACAACAACTTT-3' and reverse, 5'-CGCGTCCTTCAAACTAATGG-3' were used in PCR reactions containing 5% DMSO (Yang, Z et al., Science 314, 992-993 (2006)). DNA was denatured 5 min at 95°C, followed by 35 cycles, each 30 sec at 94°C, 30 sec at 52°C, and 45 sec at 72°C per cycle. The PCR product was digested with *Eagl* to identify the HtrA1 G allele.

### Example 3

### Analysis of frozen human brain tissue from AD patients and controls

All cases had been collected at the Institute of Neuropathology, University Hospital Muenster. Prior to autopsies, consent from patient's families was obtained to use samples for research. The neuropathological diagnosis of AD was made according to established criteria (Braak et al. Acta Neuropathol. (Berl) 82: 239-259 (1991); Mirra et al. Neurology 41:479-486 (1991)). The control group consisted of brains from cases without neurological and neuropathological abnormalities. RNA and protein was harvested from frozen post-mortem samples of the frontal gray matter of each 14 individuals with AD and 11 age matched controls. Samples were homogenised using a micro-dismembrator. Before cDNA synthesis, RNA quality was analyzed by gel electrophoresis. Only samples with acceptable RNA quality were used for cDNA synthesis (11 AD cases (mean age 76.3 years; range 63 to 90; mean post mortem time 24.6 h, range 5 to 48 h) and 10 sex matched controls (mean age 72.6 years; range 63 to 92; mean post mortem time 22.3 h, range 5 to 43 h)).

### Example 4

### Quantitative image analysis of Alzheimer plaques and neurofibrillary tangles

Quantification of Alzheimer pathology was performed using sections from the medial frontal and the medial temporal gyrus according to the methodology described before (Egensperger et al., Brain Pathology 8: 439-447 (1998)). In brief, immunophenotypes of AD brains were analyzed quantitatively by determining the number and tissue area covered by cortical Aß4-immunoreactive plaques and Aß4/tau-positive neuritic plaques, as well as the number of neurofibrillary tangles. Slides were imaged using a Leitz Aristoplan microscope with a 10X or 6.3X objective connected to a colour video camera (Sony, 3 CCD, DXC-930P) and analyzed using the computer-based image analysis system Optimas (version 5.1, Optimas Corporation, Seattle, Washington). Significance of the differences in distribution of plaques or neurofibrillary tangles between AD patients carrying the HtrA1 genotype GG and those with AG genotype was computed using the Kruskal-Wallis test. SPSS 14.0.1 (Statistical Package for the Social Sciences) was employed for the statistical analyses.

### Example 5

### HtrA1 RealTime PCR

RNA was isolated from 15 mg frontal cortex, which was homogenised in 350 µl RLT buffer (RNeasy MiniKit, Qiagen, Hilden, Germany) using an ultra turrax homogenizer. Template cDNA was synthesized from 2 µg total RNA using the high capacity cDNA archive kit (Applied Biosystems), following manufacturer's instructions. SYBR Green assays were performed on a GeneAmp 5700 Sequence Detection System (Applied Biosystems, Foster City, CA The primer pair for HtrA1 was 5'- GCAACTCAGACATGGACTACATC-3' (forward) and 5'- GTGTTAATTCCAATCACTTCACCG-3' (reverse). Cycling conditions were 50°C for 2 min, 95°C for 15 min, followed by 40 cycles of denaturation at 95°C for 15 s, and annealing and elongation at 60°C for 60 s.

The SYBR Green reagent (Applied Biosystems) was employed for the PCR product labelling and the 7500 Fast Real time PCR System (Applied Biosystems) was used for performing PCR and data collection. The total reaction volume was of 12.5 µl, containing 6.25 µl Power SYBR Green PCR Master Mix (Applied Biosystems), 1.25 µl Primer (4.5 µM each) and 1 µl cDNA. Gene expression ratios for each sample (regulation factors, standard deviation) were calculated according the ddCt method (Livak et al., Methods 25, 402-408 (2001)), and normalized against GAPDH. Experiments were performed on two independent dates in quadruplicate. Melting curve analysis confirmed that only one product was amplified. Specificity was confirmed by gel electrophoresis of PCR products showing only one product with each primer set.

### Example 6

### HtrA1 and Aß40/42 ELISA

Protein was isolated from 100 mg frontal cortex, which was homogenised in 200 µl sample buffer (50 mM Tris HCl, pH 7,4, 150 mM NaCl, 1 mM EDTA, 1 mM PMSF, protease inhibitor cocktail) using an ultra turax homogenizer. Cell lysate was centrifuged for 5 min at 13 000 rpm and the supernatant was further used for ELISA. Protein concentration was determined using with the Bradford method.

### Example 7

### Aß40/42 ELISA

For determination of Aß40 and Aß42 concentrations the Tkbrain-Set kits from the Genetics Company (Zurich, Switzerland) were used. Sample preparation was performed according to the following protocol:
Weigh human brain without thawing. Place in 15 ml tube, add 800 µl THB (for 250 ml: 21.4 g sucrose, 5 ml 1 M Tris base, 0.5 ml 0.5 M EDTA, 250 µl 0.1 M EGTA) with protease inhibitor. Homogenise the tissue by sonication (3x ) on ice (30 sec sonication, 1 min rest). Incubate on ice for 30 min. Transfer 300 µl of the homogenate to a clean tube. Add 700 µl 70% formic acid, homogenise the solution by sonication (2x) on ice (30 sec, 1 min rest). Ultracentrifuge at 100,000 for 1 h at 4°C. Carefully remove the sample from the centrifuge. There should be three layers in the tube: the upper fatty acid layer (orange), the formic acid extract containing Aß, and the debris pellet (normally very little can be observed). Transfer the middle layer to a clean tube. For 200 µl formic acid extract, use 3 ml Formic Acid Neutralization Buffer (for 500 ml: 60.57 g Tris base, 33.5 g Na₂HPO₄). Aliquot the sample and store at -80°C.

### Example 8

The HtrA1 ELISA assay was performed according to the following protocol:
Material:
   ELISA plates Immulon HBX from Thermolabsystem
   Monoclonal HtrA1 antibody
   Polyclonal HtrA1 antibody
   Swine-anti rabbit biotin conjugated (Dako Cytomation)
   Streptavidin HRP conjugated (Amersham)
   PBS tablets (Oxoid)
   TNB (3,3',5,5'Tetramethylbenzidine) (Sigma)
   BSA from Sigma
   HtrA1 Standard reference (STD) at 10 ng/ml; dilute HtrA1 to a concentration of 1 ng/ml in 1%
   BSA/PBS and make 1 ml aliquots; freeze them at -20°C or -70°C
Protocol for 1 plate:
   Prepare fresh each time:
      PBS for dilution of antibodies and BSA: dissolve 1 PBS tablet (Oxoid) in 100 ml distilled water
      5% BSA: dissolve 2.5 g of BSA in 50 ml PBS
      1% BSA: dilute 10 ml of the 5% BSA/PBS solution with 40 ml PBS
      Wash buffer (PBS/Tween): dilute 200 ml of 10xPBS buffer in 1.8 l of distilled water and add 1 ml of Tween-20. Adjust to pH 7.4 if necessary.
      Dilute 120 µl of monoclonal HtrA1 antibody in 12 ml PBS
      Mix and pipette 100 µl per well with multichannel pipette
      Cover the plate with parafilm and incubate overnight at 4°C
      Next morning
      Make up the BSA solutions
      Wash the plate 4x with 400 µl PBS-Tween
      Add 300 µl 3% BSA/PBS to each well and incubate the plate at RT for 1 h.
   STD curve:
      Use a 10 ng/ml HtrA1 stock solution
Prepare serial 1:2 dilutions in 1% BSA/PBS until reaching a concentration of 0.1 ng/ml HtrA1. STD must be kept on ice until used. After blocking, wash the plate 4x with wash buffer. Add STD in triplicates (100 µl/well). Use 100 µl 1% BSA/PBS as reference. Add samples (A-W or so) in triplicates to the wells (100 µl/well). Use the buffer or medium the samples are in as a reference.

### Example:

| STD | | | samples | | | | | | | | |
|---|---|---|---|---|---|---|---|---|---|---|---|
| 10 | 10 | 10 | A | A | A | I | | | Q | | |
| 5 | 5 | 5 | B | B | B | J | | | R | | |
| 2,5 | 2,5 | 2,5 | C | C | C | K | | | S | | |
| 1,25 | 1,25 | 1,25 | D | | | L | | | T | | |
| 0,6 | 0,6 | 0,6 | E | | | M | | | U | | |
| 0,3 | 0,3 | 0,3 | F | | | N | | | V | | |
| BSA | BSA | BSA | G | | | O | | | w | | |
| BSA | BSA | BSA | H | | | P | | | ref | ref | ref |

Cover the plate with parafilm and shake for 2 h at 30°C (200-300 rpm)
Wash the plate 4x with wash buffer
Dilute 24 µl polyclonal HtrA1 antibody in 12 ml 1% BSA/PBS (1:500 dilution)
Add 100 µl/well, cover with parafilm and incubate for 1 h shaking at 30°C
Wash the plate 4x with wash buffer
Dilute 2.4 µl swine-anti rabbit biotin conjugated antibody in 12 ml 1% BSA/PBS (1:5,000 dilution)
Add 100 µl/well, cover with parafilm and incubate for 1 h shaking at 30°C Wash the plate 4x with wash buffer
Dilute 24 µl Streptavidin-HRP in 12 ml 1% BSA/PBS (1:500 dilution)
Add 100 µl/well, cover with parafilm and incubate for 30 min shaking at RT
10 min before the incubation time is finished, weigh in 0.05 g TNB in a 15 ml plastic tube
Prepare a second tube with 12 ml Galatti buffer
Put both to 4°C
Wash the plate 4x with wash buffer
Add 1 ml DMSO to the 0.05 g TNB, vortex
Add 1 ml 100% EtOH and vortex
Add 120 µl of TNB solution to the 12 ml Galatti buffer, vortex
Add 100 µl TNB/Galatti to each well
Shake in the dark at RT (cover with saran wrap) for 10-30 min or until colour changes to blue. The intensity of the samples should be within the STD curve.
Stop the reaction by adding 50 µl stop solution (the colour will change to yellow) and read the plate immediately at 405 nm in a plate reader.
10xPBS:
   2.6 g KH₂PO₄
   21.7 g Na₂HPO₄ or NaH₂PO₄
   87.1 g NaCl
   Make up to 900 ml with distilled water
   Adjust the pH to 7.4 with HCl or NaOH and fill up to 1 l with distilled water
Galatti buffer:
   8.4 g citric acid
   Add 160 ml distilled water
   Adjust pH to 3.95 with KOH
   Make up to 200 ml with distilled water
   Add 68 µl H₂O₂
Stop reagent:
   7% H₂SO₄

### Example 9

### Purification of HtrA1

HtrA1 was cloned into a pET vector and transformed into *Escherichia coli* strain BL21 D3⁺. NZA Amp medium was inoculated and bacteria were grown for 3 hours at 37°C. Subsequently, protein production was induced with isopropyl ß-D-thiogalactoside (IPTG; 0.5 mM) and bacteria were grown further for 5 hours at 25°C.

Cells were harvested by centrifugation for 30 minutes at 4000 g at 4°C. Cell pellet was resuspended in cold lysis buffer (50 mM NaH₂PO₄, pH 7.5 / 300 mM NaCl). Cells were opened by French pressing and the lysate was cleared by an additional centrifugation step for 40 minutes at 20000 g. The supernatant was removed and loaded on a Ni²⁺-NTA Superflow column (Qiagen). To remove unbound proteins the column was washed with washing buffer l (100 mM Tris, pH 7.5 / 150 mM NaCl / 5 mM ß-mercaptoethanol) and washing buffer II (100 mM Tris, pH 7.5 / 150 mM NaCl / 5 mM ß-mercaptoethanol / 30 mM imidazol). Protein was eluted with elution buffer (100 mM Tris, pH 7.5 / 150 mM NaCl / 5 mM ß-mercaptoethanol / 150 mM imidazole). Fractions containing HtrA1 were pooled and loaded on a Hydroxyapatite (HPS) column (BioRad).

The HPS column was washed with starting buffer (50 mM HEPES, pH 8 / 50 mM NaCl). Then, a gradient of starting buffer and end buffer (50 mM HEPES pH 8 / 50 mM NaCl / 500 mM K₂HPO₄ and KH₂PO₄) was loaded on the column. Fractions containing pure HtrA1 were pooled. HtrA1 was dialysed against dialyses buffer (50 mM Tris-HCl, pH 8 / 150 mM NaCl / 10% glycerol) and stored in aliquots at -80°C.

### Medium :

### NZA-(NZ-AmineA)

10 g/l NZ-AmineA
5 g/l Bacto-Yeast-Extract
7.5 g/l NaCl
in dH₂O

### Example 10

### Purification of tau proteins

Tau constructs (tau-wt, tau-Ala and tau-PHP) were expressed from a pET vector. Transformed bacteria were grown as described in Example 9 and induced with IPTG (1 mM) and grown for 3 hours at 42°C. Cells harvested by centrifugation for 10 minutes at 10000 g at 4°C. The cell pellet was resuspended in cold tau-lysis buffer (33 mM Tris-HCl, pH 8 / 100 mM KCI). Cell lysate was prepared by French pressing and cleared by centrifugation for 40 minutes at 13000 g. The supernatant was boiled for 20 minutes followed by an additional centrifugation step for 40 minutes at 35000 g. The supernatant was precipitated with 30% ammonium sulphate. The ammonium sulphate solution was shaken for 30 minutes at 4°C followed by a centrifugation step for 30 minutes at 20000 g. The supernatant was transferred in a clean tube and ammonium sulphate was added to a final concentration of 40%. The solution was shaken for 30 minutes at 4°C, followed by centrifugation for 30 minutes at 20000 g. The pellet was resuspended in storage-buffer (80 mM PIPES-KOH, pH 6.8 / 1 mM EGTA / 1 mM CaCl₂) and aliquots were stored at -80°C.

### Example 11

### Digestion of tau by HtrA1

Three different tau proteins (tau-wt, tau-Ala and tau-PHP) were incubated with HtrA1 at an enzyme:substrate ratio of 1:2. The reaction mixture contained 50 mM Tris-HCl, pH 8. Aliquots of the samples were removed at various time points and mixed immediately with 5 x sample buffer containing DTT and boiled immediately for 5 minutes at 99°C to terminate the digestion. The samples were used for gel electrophoresis and immunoblotting. Antibodies used were rabbit polyclonal antibody against HtrA1 generated against the HtrA1-PDZ domain comprising amino acids 365-467. Polyclonal antibody against tau was from ZYMED Laboratories.

### Electrophoresis and blotting

Aliquots of the digested samples, each containing 2 µg of tau, were subjected to 12% SDS-PAGE. Proteins were stained with Coomassie Brilliant Blue. For immunoblotting, 1 µg of tau protein was loaded. Proteins were electrotransferred to nitrocellulose, and incubated with 5% milk powder in Tris buffered saline (TBS) complemented with 0.1% Tween, to block nonspecific binding. Subsequently, the blot was incubated with anti-tau antibody (1:10000 diluted in 5% milk powder TBS-T). As secondary antibody an anti-mouse antibody (conjugated to alkaline phosphatase, diluted 1:20000 in 5% milk powder TBS-T) was added and proteins were visualised with NBT/BCIP mixture in AP buffer.

### Buffer:

### TBS-T 0.1%

20 mM Tris-HCl, pH 7.5
150 mM NaCl
1 ml Tween
in dH₂O

### AP buffer

100 mM Tris-HCl, pH 9.5
100 mM NaCl
5 mM MgCl₂
in dH₂O

### Example 12

### Tau aggregation in the presence of formaldehyde

4 µg of purified tau proteins (tau-wt, tau-Ala and tau-PHP) were incubated with 1% formaldehyde in 50 mM NaH₂PO₄, pH 7.5. Tau was incubated at 37°C overnight to allow aggregation to take place. Protein was precipitated with 6% Tri-Chloric-Acid (TCA), centrifuged for 5 minutes at 13000 g and the pellet was resuspended in 50 mM Tris-HCl, pH 8

### Example 13

### Digestion of aggregated tau

HtrA1 was added to aggregated and crosslinked tau proteins. The samples were incubated overnight at 37°C. As a control, aggregated tau and HtrA1 was incubated separately overnight at 37°C. Subsequently, samples were loaded on a Tris-Tricine gel.

### Example 14

### HtrA1 expression levels in PC12 cells expressing various tau constructs

### Cell culture

The cell line PC12, derived from rat medulla, was cultured in DMEM supplemented with 10% fetal bovine serum, 5% horse serum, 1% penicillin-streptomycin and G418 (1:200). Cells were grown in six-well plates to 80% confluence.

### RNA extraction and Q-RT-PCR

5 x 10⁵ PC12 cells were seeded into a six-well plate. After two days the six-well plate was 80% confluent and RNA was extracted by using total RNA isolation kit (Macherey-Nagel). RNA was purified with NucleSpin RNAII columns (Macherey-Nagel) according to the procedure by the manufacturer. 1 µg of pooled RNA was used for cDNA synthesis by Verso^{™} cDNA kit (Thermo Scientific); the cDNA synthesis was performed as described by the manufacturer.

Quantitative RT-PCR was done with Platinum SYBR Green qPCR-superMix-UDG-kit (Invitrogen) with Rotor-Gene RG3000 from Corbett Research. The following oligonucleotides were used:
rat-HtrA1-forward-GATGTAATCTCCGGAGCATATATC;
rat-HtrA1-reverse-CCTTTTTGATGACATCACTGACATC;
rat-ß-actin-forward-GATTACTGCTCTGGCTCCTAG;
rat-ß-actin-reverse-ACTCATCGTACTCCTGCTTGC.

The total reaction volume was of 25 µl, containing 12.5 µl SYBR Green PCR Master Mix (Invitrogen), 1 µl Primer (4.5 µM each) and 2 µl cDNA (20 ng). The following cycling conditions were used, 95°C for 15 minutes, followed by 40 cycles of denaturation at 95°C for 15 s, annealing at 55°C for 30 s and elongation at 72°C for 30 s. Melting curve was measured every 5 seconds from 50°C to 99°C. Gene expression ratios for each sample were calculated as described (Pfaffl, *Nucleic Acids Res,* 29:e45 (2001)). Melting curve analysis confirmed that only one product was amplified.

### Example 15

### Identification of tau fragments produced by HtrA1 digests using Mass Spectrometry

Tau proteins (tau-wt, tau-Ala and tau-PHP; 100 µg) were incubated with HtrA1 in reaction buffer 50 mM Tris-HCl, pH 8. After incubation overnight at 37°C, samples were precipitated by acetone. 100 µl of sample was mixed with 700 µl acetone and incubated overnight at-20°C. The next day, samples were centrifugation for 10 minutes at 13000 g. The supernatant was removed, concentrated by speed Vac (Eppendorf Concentrator 5301) and analysed by standard MALDI-TOF Mass Spectrometry.

### Example 16

### Identification of peptidic activators

HtrA1 interacts with presenilin 1 (PS1). This was evident from data obtained from copurification, comigration during size exclusion chromatography and two hybrid analyses. Therefore a peptide comprised of the 10 C-terminal residues of PS1 (DQLAFHQFYI) was synthesized and shown to activate HtrA1 as shown by a more efficient degradation of tau (see Fig 18). Such a peptide could be used as a basis to synthesise a potent HtrA1 activator that could be of therapeutic relevance. A second peptide KKKDSRIWWV turned out to be a weaker activator. This peptide was derived from a study of Sidhu et al (Runyon et al. Protein Sci. 16:2454-71 (2007)) who reported excellent binding of DSRIWWV to the PDZ domain of HtrA1. This peptide did however not bind to HtrA1 composed of protease and PDZ domain as shown by Isothermal Titration Calorimetry. Only after adding 3 N-terminal residues, the peptide did bind to HtrA1.

### Example 17

### Composition of non-peptidic chemical activators

Non-peptidic PDZ ligands as activators can be composed of a central heterocyclus serving as a platform (e.g. indoles, quinolines, benzothiophens). One end of the platform contains a COOH group (or other negatively charged groups), mimicking the C-terminus of a peptidic ligand. This COOH group interacts with the conserved Arg residue at the end of the binding pocket of the PDZ domain. In the vicinity of this terminal carboxylate group a methyl (or similar) group mimicking the P1, 2 or 3 residue of the PDZ ligand (depending on the position and thus the distance to the COOH group) stabilises the interaction of the ligand with the PDZ domain and provide specificity. Additional groups containing e.g. aromatic structures can follow or be placed in between to reach over into the protease domain where it interacts with loops or other structures of the protease domain for the purpose of activation. Similarly inhibitors can be designed. Here structures protruding from the PDZ to the protease domains that unproductively interact with elements of the protease domain (including but not restricted to the active site or other elements like loop 3 or the S1 specificity pocket) are expected to act as specific reversible or irreversible inhibitors of enzymatic function.

### References

Selkoe DJ. Alzheimer's disease: genes, proteins, and therapy. Physiol Rev. 2001 81:741-66.
Haass C, Schlossmacher MG, Hung AY, Vigo-Pelfrey C, Mellon A, Ostaszewski BL, Lieberburg I, Koo EH, Schenk D, Teplow DB, et al. Amyloid beta-peptide is produced by cultured cells during normal metabolism. Nature. 1992 359:322-5.
Seubert P, Oltersdorf T, Lee MG, Barbour R, Blomquist C, Davis DL, Bryant K, Fritz LC, Galasko D, Thal LJ, et al. Secretion of beta-amyloid precursor protein cleaved at the amino terminus of the beta-amyloid peptide. Nature. 1993 361:260-3.
Czech C, Tremp G, Pradier L. Presenilins and Alzheimer's disease: biological functions and pathogenic mechanisms. Prog Neurobiol. 2000 60:363-84
Hutton M, Hardy J. The presenilins and Alzheimer's disease. Hum Mol Genet. 1997; 6:1639-46.
Tanzi RE, Moir RD, Wagner SL. Clearance of Alzheimer's Abeta peptide: the many roads to perdition. Neuron. 2004; 43:605-8.
Grau S, Baldi A, Bussani R, Tian X, Stefanescu R, Przybylski M, Richards P, Jones SA, Shridhar V, Clausen T, Ehrmann M. Implications of the serine protease HtrA1 in amyloid precursor protein processing. Proc Natl Acad Sci U S A. 2005 102:6021-6
Avila J. Tau phosphorylation and aggregation in Alzheimer's disease pathology. FEBS Lett. 2006; 580:2922-7
Goate A, Chartier-Harlin MC, Mullan M, Brown J, Crawford F, Fidani L, Giuffra L, Haynes A, Irving N, James L, et al. Segregation of a missense mutation in the amyloid precursor protein gene with familial Alzheimer's disease. Nature. 1991; 349:704-6.
Sherrington R, Rogaev El, Liang Y, Rogaeva EA, Levesque G, Ikeda M, Chi H, Lin C, Li G, Holman K, et al. Cloning of a gene bearing missense mutations in early-onset familial Alzheimer's disease. Nature. 1999; 375:754-60.
Levy-Lahad E, Wasco W, Poorkaj P, Romano DM, Oshima J, PettingeII WH, Yu CE, Jondro PD, Schmidt SD, Wang K, et al. Candidate gene for the chromosome 1 familial Alzheimer's disease locus. Science. 1995; 269:973-7.
Rogaev El, Sherrington R, Rogaeva EA, Levesque G, Ikeda M, Liang Y, Chi H, Lin C, Holman K, Tsuda T, et al. Familial Alzheimer's disease in kindreds with missense mutations in a gene on chromosome 1 related to the Alzheimer's disease type 3 gene. Nature. 1995; 376:775-8.
Saunders AM, Strittmatter WJ, Schmechel D, George-Hyslop PH, Pericak-Vance MA, Joo SH, Rosi BL, Gusella JF, Crapper-MacLachlan DR, Alberts MJ, et al. Association of apolipoprotein E allele epsilon 4 with late-onset familial and sporadic Alzheimer's disease. Neurology. 1993; 43:1467-72.
Schmechel DE, Saunders AM, Strittmatter WJ, Crain BJ, Hulette CM, Joo SH, Pericak-Vance MA, Goldgaber D, Roses AD. Increased amyloid beta-peptide deposition in cerebral cortex as a consequence of apolipoprotein E genotype in late-onset Alzheimer's disease. Proc Natl Acad Sci U S A. 1993; 90:9649-53.
Strittmatter WJ, Saunders AM, Schmechel D, Pericak-Vance M, Enghild J, Salvesen GS, Roses AD. Apolipoprotein E: high-avidity binding to beta-amyloid and increased frequency of type 4 allele in late-onset familial Alzheimer's disease. Proc Natl Acad Sci U S A. 1993; 90:1977-81.
Corder EH, Saunders AM, Strittmatter WJ, Schmechel DE, Gaskell PC, Small GW, Roses AD, Haines JL, Pericak-Vance MA. Gene dose of apolipoprotein E type 4 allele and the risk of Alzheimer's disease in late onset families. Science. 1993; 261:921-3.
Slooter AJ, Cruts M, Kalmijn S, Hofman A, Breteler MM, Van Broeckhoven C, van Duijn CM. Risk estimates of dementia by apolipoprotein E genotypes from a population-based incidence study: the Rotterdam Study. Arch Neurol. 1998; 55:964-8.
Daw EW, Payami H, Nemens EJ, Nochlin D, Bird TD, Schellenberg GD, Wijsman EM. The number of trait loci in late-onset Alzheimer's disease. Am J Hum Genet. 2000; 66:196-204.
Bertram L, Blacker D, Mullin K, Keeney D, Jones J, Basu S, Yhu S, McInnis MG, Go RC, Vekrellis K, Selkoe DJ, Saunders AJ, Tanzi RE. Evidence for genetic linkage of Alzheimer's disease to chromosome 10q. Science. 2000; 290:2302-3.
Blacker D, Bertram L, Saunders AJ, Moscarillo TJ, Albert MS, Wiener H, Perry RT, Collins JS, Harrell LE, Go RC, Mahoney A, Beaty T, Fallin MD, Avramopoulos D, Chase GA, Folstein MF, McInnis MG, Bassett SS, Doheny KJ, Pugh EW, Tanzi RE; NIMH Genetics Initiative Alzheimer's Disease Study Group. Results of a high-resolution genome screen of 437 Alzheimer's disease families. Hum Mol Genet. 2003; 12:23-32.
Myers A, Wavrant De-Vrieze F, Holmans P, Hamshere M, Crook R, Compton D, Marshall H, Meyer D, Shears S, Booth J, Ramic D, Knowles H, Morris JC, Williams N, Norton N, Abraham R, Kehoe P, Williams H, Rudrasingham V, Rice F, Giles P, Tunstall N, Jones L, Lovestone S, Williams J, Owen MJ, Hardy J, Goate A. Full genome screen for Alzheimer's disease: stage II analysis. Am J Med Genet. 2002; 114:235-44.
Mirra SS, Heyman A, McKeel D, Sumi SM, Crain BJ, Brownlee LM, Vogel FS, Hughes JP, van Belle G, Berg L. The Consortium to Establish a Registry for Alzheimer's Disease (CERAD). Part II. Standardization of the neuropathologic assessment of Alzheimer's disease. Neurology. 1991; 41:479-86.
Bertram L, Tanzi RE. Alzheimer's disease: One disorder, too many genes? Hum Mol Genet 2004; 13 Spec No 1:R135-141
Li YJ, Scott WK, Hedges DJ, Zhang F, Gaskell PC, Nance MA, Watts RL, Hubble JP, Koller WC, Pahwa R, Stern MB, Hiner BC, Jankovic J, Allen FA Jr, Goetz CG, Mastaglia F, Stajich JM, Gibson RA, Middleton LT, Saunders AM, Scott BL, Small GW, Nicodemus KK, Reed AD, Schmechel DE, Welsh-Bohmer KA, Conneally PM, Roses AD, Gilbert JR, Vance JM, Haines JL, Pericak-Vance MA. Age at onset in two common neurodegenerative diseases is genetically controlled. Am J Hum Genet. 2002; 70:985-93
Ozturk A, Desai PP, Minster RL, Dekosky ST, Kamboh MI. Three SNPs in the GSTO1, GSTO2 and PRSS11 genes on chromosome 10 are not associated with age-at-onset of Alzheimer's disease. Neurobiol Aging. 2005; 26:1161-5
Abou-Sleiman PM, Muqit MM, Wood NW. Expanding insights of mitochondrial dysfunction in Parkinson's disease. Nat Rev Neurosci. 2006; 7:207-19.
Clausen T, Southan C, Ehrmann M. The HtrA family of proteases: implications for protein composition and cell fate. Mol Cell. 2002; 10:443-55.
Nie G, Li Y, He H, Findlay JK, Salamonsen LA. HtrA3, a serine protease possessing an IGF-binding domain, is selectively expressed at the maternal-fetal interface during placentation in the mouse. Placenta. 2006; 27:491-501
Zumbrunn J, Trueb B. Primary structure of a putative serine protease specific for IGF-binding proteins. FEBS Lett. 1996 ;398:187-92.
Shridhar V, Sen A, Chien J, Staub J, Avula R, Kovats S, Lee J, Lillie J, Smith DI. Identification of underexpressed genes in early- and late-stage primary ovarian tumors by suppression subtraction hybridization. Cancer Res. 2002; 62:262-70.
Baldi A, De Luca A, Morini M, Battista T, Felsani A, Baldi F, Catricala C, Amantea A, Noonan DM, Albini A, Natali PG, Lombardi D, Paggi MG. The HtrA1 serine protease is downregulated during human melanoma progression and represses growth of metastatic melanoma cells. Oncogene. 2002 ;21:6684-8.
Grau S, Richards PJ, Kerr B, Hughes C, Caterson B, Williams AS, Junker U, Jones SA, Clausen T, Ehrmann M. The role of human HtrA1 in arthritic disease. J Biol Chem. 2006; 281:6124-9.
Hageman GS, Anderson DH, Johnson LV, Hancox LS, Taiber AJ, Hardisty LI, Hageman JL, Stockman HA, Borchardt JD, Gehrs KM, Smith RJ, Silvestri G, Russell SR, Klaver CC, Barbazetto I, Chang S, Yannuzzi LA, Barile GR, Merriam JC, Smith RT, Olsh AK, Bergeron J, Zernant J, Merriam JE, Gold B, Dean M, Allikmets R. A common haplotype in the complement regulatory gene factor H (HF1/CFH) predisposes individuals to age-related macular degeneration. Proc Natl Acad Sci U S A. 2005; 102:7227-32.
Dewan A, Liu M, Hartman S, Zhang SS, Liu DT, Zhao C, Tam PO, Chan WM, Lam DS, Snyder M, Barnstable C, Pang CP, Hoh J. HTRA1 promoter polymorphism in wet age-related macular degeneration. Science. 2006; 314:989-92.
Yang Z, Camp NJ, Sun H, Tong Z, Gibbs D, Cameron DJ, Chen H, Zhao Y, Pearson E, Li X, Chien J, Dewan A, Harmon J, Bernstein PS, Shridhar V, Zabriskie NA, Hoh J, Howes K, Zhang K. A variant of the HTRA1 gene increases susceptibility to age-related macular degeneration. Science. 2006; 314:992-3.
Daw EW, Heath SC, Wijsman EM. Multipoint oligogenic analysis of age-at-onset data with applications to Alzheimer's disease pedigrees. Am J Hum Genet. 1999; 64:839-51.
Duggirala R, Blangero J, Almasy L, Dyer TD, Williams KL, Leach RJ, O'Connell P, Stern MP. Linkage of type 2 diabetes mellitus and of age at onset to a genetic location on chromosome 10q in Mexican Americans. Am J Hum Genet. 1999; 64:1127-40.
Livak KJ, Schmittgen TD. Analysis of relative gene expression data using real-time quantitative PCR and the 2(-Delta Delta C(T)) Method. Methods 2001; 25:402-408.
McKhann G, Drachman D, Folstein M, Katzman R, Price D, Stadlan EM. Clinical diagnosis of Alzheimer's disease: report of the NINCDS-ADRDA Work Group under the auspices of Department of Health and Human Services Task Force on Alzheimer's disease. Neurology 1983; 34:939-944.
Braak H, Alafuzoff I, Arzberger T, Kretzschmar H, Del Tredici K. Staging of Alzheimer disease-associated neurofibrillary pathology using paraffin sections and immunocytochemistry. Acta Neuropathol (Berl). 2006; 112:389-404.
Egensperger R, Kosel S, von Eitzen U, Graeber MB. Microglial activation in Alzheimer's disease: Association with APOE genotype. Brain Pathol. 1998; 8:439-47.
Botstein D., White RL, Skolnick M, Davis RW. Construction of a genetic linkage map in man using restriction fragment length polymorphisms. Am J Hum Genet. 1980; 32(3):314-31.
Riesner D., Steger G, Wiese U, Wulfert M, Heibey M, Henco K. Temperature-gradient gel electrophoresis for the detection of polymorphic DNA and for quantitative polymerase chain reaction. Electrophoresis. 1992; 13(9-10):632-6.
Hayashi J, Ohta S, Kikuchi A, Takemitsu M, Goto Y, Nonaka I. Introduction of disease-related mitochondrial DNA deletions into HeLa cells lacking mitochondrial DNA results in mitochondrial dysfunction. Proc Natl Acad Sci. 1991; 88(23):10614-8.
Keen J., Lester D., Inglehearn C., Curtis A, and Bhattacharya S. Rapid detection of single base mismatches as heteroduplexes on Hydrolink gels. Trends Genet. 1991; 7:5.
Grompe M. The rapid detection of unknown mutations in nucleic acids. Nat. Genet. 1993; 5(2):111-7.
Sanger F, Nicklen S, Coulson AR. DNA sequencing with chain-terminating inhibitors. Proc Natl Acad Sci USA. 1977; 74(12):5463-7.
Ronaghi M, Uhlen M, Nyren P. A sequencing method based on real-time pyrophosphate. Science. 1998; 281 (5375):363, 365.
Ronaghi M. Pyrosequencing sheds light on DNA sequencing. Genome Res.. 2001; 11(1):3-11.
Ahmadian A, Gharizadeh B, Gustafsson AC, Sterky F, Nyren P, Uhlen M, Lundeberg J. Single-nucleotide polymorsphism analysis by pyrosequencing. Anal Biochem. 2000; 10; 280(1):103-10.
Tyagi, S, F.R. Kramer. Molecular beacons: Probes that fluoresce upon hybridization. Nature Biotechnol. 1996; 14:303-308.
Tyagi, S,. D.P. Bratu, F.R. Kramer. Multicolor molecular beacons for allele discrimination. Nature Biotechnol. 1998; 16:49-53.
Howell WM, Jobs M, Gyllensten U, Brookes AJ. Dynamic allele-specific hybridization. A new method for scoring single nucleotide polymorphisms. Nat Biotechnol. 1999; 17(1):87-8.
Newton, C.R., Graham A, Heptinstall L.E., Powell S.J., Summers C., Kalsheker N., Smith J.C., Markham A.F. Analysis of any point mutation in DNA. The amplification refractory mutation system (ARMS). Nucleic Acids Res. 1989; 17:2503-2516.
Sommers, S.S., Cassady J. D., Sobell J. L., Bottema, C. D. K. A novel method for detecting point mutations or polymorphisms and its application to population screening for carriers of phenylketonuria. Mayo Clin. Proc. 1989; 64:1361-1372.
Liu Y., Phelan J., Go R.C., Prchal J.F., Prchal J. T. Rapid determination of clonality by detection of two closely-linked X chromosome exonic polymorphisms using allele-specific PCR. J Clin Invest. 1997; 99(8):1984-90.
Hacia, J. G. Resequencing and mutational analysis using oligonucleotide microarrays. Nature Genet., 1999a; 21, 42-47.
Hacia, J. G., Fan, J. B., Ryder, O., Jin, L., Edgemon, K., Ghandour, G., Mayer, R. A., Sun, B., Hsie, L., Robbins, C. M. Determination of ancestral alleles for human single-nucleotide polymorphisms using high-density oligonucleotide arrays. Nature Genet. 1999b; 22, 164-167.
Pease, A., C., D. Solas, Sullivan E. J., Cronin M. T., C. P. Holmes & S. P. A. Fodor. Light-generated oligonucleotide arrays for rapid DNA sequence analysis. Prog. Natl. Acad. Sci. USA 1994; 91:5022-5026.
Sosnowski, R. G., Tu, E., Butler, W. F., O'Connel, J. P., Heller, M. J. Rapid determination of single base mismatch mutations in DNA hybrids by direct electric field control. Proc. Natl. Acad. Sci. USA 1997; 94, 1119-1123.
Gilles, P. N., Wu, D. J., Foster, C. B., Dillon, P. J., Chanock, S. J. Single nucleotide polymorphic discrimination by an electronic dot blot assay on semiconductor microchips. Nat Biotechnol. 1999, Apr.; 17(4):365-70.
Nguyen, H. K., Fournier, O., Asseline, U., Dupret, D., Nguyen, T. T. Smoothing of the thermal stability of DNA duplexes by using modified nucleosides and chaotropic agents. Nucleic Acids Res. 1999; 27, 1492-1498.
Landegren, U., Kaiser, R., Sanders, J., Hood, L. A ligase-mediated gene detection technique. Science 1988; 241:1077-1080.
Wu, D. Y., Wallace R. B. Specificity of the nick-closing activity of bacteriophage T4 DNA ligase. Gene 1989; 76:245-254.
Parik, J., Kwiatkowski, M., Lagerkvist, A., Lagerström-Fermér, M., Samiotaki, M., Stewart, J., Glad, G., Mendel-Hartvig, M., Landegren, U. A manifold support for molecular genetic reactions. Anal. Biochem. 1993; 211:144-150.
Samiotaki, M., Kwiatkowski, M., Parik, J., Landegren, U. Dual-color detection of DANN sequence variants by ligase-mediated analysis. Genomics 1994; 20:238-242.
Grossmann, P. D., Bloch, W., Brinson, E., Chang, C. C., Eggerding, F. A., Fung, S., lovannisci, D. A., Woo, S., Winn-Deen, E. S. High-density multiplex detection of nucleic acid sequences: Oligonucleotide ligation assay and sequence-coded separation. Nucleic Acids Res. 1994; 22:4527-4534.
Day, D. J., Speiser, P. W., White, P. C., Barany, F. Detection of steroid 21-hydroxylase alleles using gene-specific PCR and a multiplexed ligation detection reaction. Genomics 1995; 29:152-162.
Lou, J., Bergstrom, D. E., Barany, F., Improving the fidelity of Thermus thermophilus DANN ligase. Nucleic Acids Res. 1996; 24:3071-3078.
Gunderson, K. L., Huang, X. C., Morris, M. S., Lipshutz, R. J., Lockhart, D. J., Chee, M. S. Mutation detection by ligation to complete n-mer DNA arrays. Genome Res,. 1998; 8, 1142-1153.
Syvänen, A. C., Aalto-Setälä, K., Harju, L., Kontula, K., Söderlund, H. A primer-guided nucleotide incorporation assay in the genotyping of apolipoprotein E. Genomics 1990; 8:684-692.
Pastinen, T., Kurg, A., Metspalu, A., Peltonen, L., Syvänen, A. C. Minisequencing: a specific tool for DNA analysis and diagnostics on oligonucleotide arrays. Genome Res. 1997; 7, 606-614.
Pastinen, T., Raition, M., Lindroos, K., Tainola, P., Peltonen, L., Syvänen, A. C. A system for specific, high-throughput genotyping by allele-specific primer extension on microarrays. Genome Res. 2000, 10, 1031-1042.
Dubiley, S., Kirillov, E., Mirzabekov, A. Polymorphism analysis and gene detection by minisquencing on an array of gel-immobilized primers. Nucleic Acids Res. 1999; 27, e19.

## Claims

1. Method of screening a subject for at least one risk factor associated with a tauopathy, comprising detecting the presence or absence of at least one risk marker in the HtrA1 gene (PRSS11) which is linked to said tauopathy, wherein the risk marker is a polymorphism or mutation of the HtrA1 gene.

2. Method of claim 1, wherein said risk marker is at least one mutation or polymorphism located in a regulatory element of the HtrA1 gene, in particular located in the promoter of the HtrA1 gene.

3. Method according to claim 2, wherein the single nucleotide polymorphism (SNP) rs11200638 is detected.

4. Method according to at least one of the claims 1 to 3, wherein said detecting step is carried out by determining the presence or absence of at least one risk marker in the HtrA1 gene by analysing the nucleic acid in a biological sample containing nucleic acids collected from said subject, wherein the risk marker, in particular a polymorphism, is preferably detected by a method selected from the group consisting of restriction fragment length polymorphisms (RFLP), temperature gradient gel electrophoresis (DGGE), single strand conformation polymorphisms (SSCP), heteroduplex analyses (HD), DNA sequencing, pyrosequencing, typing using molecular beacons, dynamic allele specific hybridisation (DASH), amplification refractory mutation system (ARMS) and allele specific hybridisation, in particular on microarrays, ELISA.

5. Method according to claim 4, wherein detection occurs by RFLP using a restriction enzyme detecting the single nucleotide polymorphism (SNP) rs11200638, in particular detecting a nucleic acid sequence comprising nucleotides selected from the nucleotides CGGCCG to identify the marked HtrA1 G allele, in particular Eagl, BsiEI, EaeI, Gdill, Sfil and BglI and their respective isoschizomers, or detecting a nucleic acid sequence comprising nucleotides selected from the nucleotides CAGCCG, to identify the marked HtrA1 A allele; the method preferably comprising the following steps:
a. Amplifying at least part of the HtrA1 gene of the subject sample using suitable oligonucleotide primers,
b. Incubating said amplified nucleic acid with a suitable restriction enzyme for detecting the respective risk marker, in particular the single nucleotide polymorphism (SNP) rs11200638,
c. Determining the size of the resultant restriction fragments, thereby analysing whether the subject carries a risk factor for said tauopathy, including the risk of age at onset (AAO).

6. Method according to at least one of the preceding claims 1 to 5, having one or more of the following characteristics:
(i) the following oligonucleotide primers are used for amplification:
Forward primer: 5'-ATGCCACCCACAACAACTTT-3'
Reverse primer 5'-CGCGTCCTTCAAACTAATGG-3';
(ii) the presence or absence of at least one or more additional risk markers linked to said tauopathy is detected, wherein said at least one additional risk marker preferably detects the presence or absence of at least one mutation or polymorphism within the ApoE gene;
(iii) the occurrence of an allelic variant associated with the risk of developing or expediting said tauopathy is detected.

7. Method according to at least one of the claims 1 to 6, wherein said tauopathy is Alzheimer's disease.

8. Use of
(i) a diagnostic kit comprising at least one reagent for detecting a risk marker as described in at least one of the claims 1 to 7, or
(ii) an array comprising a substrate carrying one or more reagents for identifying in a nucleic acid sample from a subject the occurrence of a risk marker in the HtrA1 gene that is associated with the risk of developing or expediting a tauopathy, wherein the risk marker is a mutation or polymorphism of the HtrA1 gene,
for determining the presence or absence of at least one risk factor as described in at least one of the claims 1 to 7 for developing or expediting a tauopathy, wherein the use is for performing the method according to at least one of the claims 1 to 7.

9. Use according to claim 8, having one or more of the following characteristics:
(i) the kit comprises at least one of the following components:
a. primers for amplifying at least part of the HtrA 1 gene, in particular primers for amplifying at least part of the promoter region of the HtrA1 gene encompassing the site of the single nucleotide polymorphism (SNP) rs11200638;
b. at least one enzyme for nucleic acid amplification;
c. at least one enzyme suitable for performing a RFLP analysis for identifying at least one risk marker;
(ii) said reagent of the array detects the single nucleotide polymorphism (SNP) rs11200638 of the HtrA1 gene;
(iii) said tauopathy is Alzheimer's disease.

10. A compound enhancing HtrA1 enzyme activity for use in a medicament for proteolysing or degrading tau, phosphorylated tau, hyperphosphorylated tau, and/or tau aggregates, phosphorylated tau aggregates and/or hyperphosphorylated tau aggregates; wherein the compound comprises or consists of a sequence selected from the group consisting of:
a. DQLAFHQFYI
b. XXXDSRIWWV, wherein X is a natural or unnatural amino acid
c. KKKDSRIWWV;
wherein the medicament is for the treatment or prevention of a tauopathy.

11. Compound according to claim 10, wherein said compound activates or stabilises the enzyme HtrA1.

12. In vitro use of HtrA1 or a functional variant thereof having HtrA1 activity for proteolysing or degrading tau, phosphorylated tau, hyperphosphorylated tau, and/or tau aggregates, phosphorylated tau aggregates and/or hyperphosphorylated tau aggregates.

13. A compound enhancing HtrA1 enzyme activity as defined in claim 10 or 11, for use in the treatment or prevention of Alzheimer's disease.

14. In vitro use of an HtrA1 activator for promoting proteolysis or degradation of tau, phosphorylated tau, hyperphosphorylated tau, and/or tau aggregates, phosphorylated tau aggregates and/or hyperphosphorylated tau aggregates; wherein the HtrA1 activator comprises or consists of a sequence selected from the group consisting of:
a. DQLAFHQFYI
b. XXXDSRIWWV, wherein X is a natural or unnatural amino acid
c. KKKDSRIWWV.

15. Use of HtrA1 in a screening assay for identifying a compound capable of inhibiting or promoting proteolysis or degradation of tau, phosphorylated tau, hyperphosphorylated tau, and/or tau aggregates, phosphorylated tau aggregates and/or hyperphosphorylated tau aggregates.

## Patentansprüche

1. Verfahren zum Untersuchen einer Testperson auf wenigstens einen mit einer Tauopathie in Verbindung stehenden Risikofaktor, umfassend das Detektieren der Anwesenheit oder Abwesenheit von wenigstens einem Risikomarker im HtrA1-Gen (PRSS11), der mit dieser Tauopathie verbunden ist, wobei der Risikomarker ein Polymorphismus oder eine Mutation des HtrA1-Gens ist.

2. Verfahren nach Anspruch 1, wobei der Risikomarker wenigstens eine Mutation oder wenigstens ein Polymorphismus ist, der in einem regulatorischen Element des HtrA1-Gens vorliegt, insbesondere im Promoter des HtrA1-Gens vorliegt.

3. Verfahren nach Anspruch 2, wobei der Einzelnukleotidpolymorphismus (Single Nucleotide Polymorphism, SNP) rs11200638 detektiert wird.

4. Verfahren nach wenigstens einem der Ansprüche 1 bis 3, wobei der Detektionsschritt ausgeführt wird durch die Bestimmung der Anwesenheit oder Abwesenheit wenigstens eines Risikomarkers im HtrA1-Gen durch Analysieren der Nukleinsäure in einer Nukleinsäuren enthaltenden biologischen Probe, die von der Testperson gesammelt wurde, wobei der Risikomarker, insbesondere ein Polymorphismus, bevorzugt durch ein Verfahren detektiert wird, das ausgewählt ist aus der Gruppe bestehend aus Restriktionsfragmentlängenpolymorphismus (RFLP), Temperaturgradientengelelektrophorese (DGGE), Einzelstrangkonformationspolymorphismus ("single strand conformation polymorphism", SSCP), Heteroduplexanalyse (HD), DNA-Sequenzierung, Pyrosequenzierung, Typisierung mit molekularen Signalen, dynamische allelspezifische Hybridisierung (DASH), Amplification Refractory Mutation System, (ARMS) und allelspezifische Hybridisierung, insbesondere auf Mikroarrays, ELISA.

5. Verfahren nach Anspruch 4, wobei die Detektion durch RFLP mit einem Restriktionsenzym stattfindet, das den Einzelnukleotidpolymorphismus (SNP) rs11200638 detektiert, insbesondere eine Nukleinsäuresequenz detektiert, die Nukleotide ausgewählt aus den Nukleotiden CGGCCG zur Identifikation des markierten HtrA1-G-Allels umfasst, insbesondere Eagl, BsiEI, Eael, Gdill, Sfil und BglI und ihre entsprechenden Isoschizomere, oder eine Nukleinsäuresequenz detektiert, die Nukleotide ausgewählt aus den Nukleotiden CAGCCG zur Identifikation des markierten HtrA1-A-Allels umfasst, wobei das Verfahren vorzugsweise die folgenden Schritte umfasst:
a. Amplifikation wenigstens eines Teils des HtrA1-Gens der Testpersonenprobe mit geeigneten Oligonukleotid-Primern,
b. Inkubieren der amplifizierten Nukleinsäure mit einem geeigneten Restriktionsenzym zur Detektion des entsprechenden Risikomarkers, insbesondere des Einzelnukleotidpolymorphismus (SNP) rs11200638,
c. Bestimmen der Größe der resultierenden Restriktionsfragmente, wodurch analysiert wird, ob die Testperson einen Risikofaktor für die Tauopathie trägt, einschließlich des Risikos des Alters bei Beginn ("age at onset", AAO).

6. Verfahren nach wenigstens einem der vorhergehenden Ansprüche 1 bis 5, mit einer oder mehrerer der folgenden Eigenschaften:
(i) die folgenden Oligonukleotid-Primer werden für die Amplifikation verwendet:
Vorwärtsprimer 5'-ATGCCACCCACAACAACTTT-3'
Rückwärtsprimer 5'-CGCGTCCTTCAAACTAATGG-3';
(ii) Die Anwesenheit oder Abwesenheit von wenigstens einem oder mehreren zusätzlichen mit der Tauopathie verbundenen Risikomarkern wird detektiert, wobei der wenigstens eine zusätzliche Risikomarker bevorzugt die Anwesenheit oder Abwesenheit von wenigstens einer Mutation oder einem Polymorphismus im ApoE-Gen detektiert;
(iii) das Auftreten einer allelischen Variante, die mit dem Risiko der Entwicklung oder Beschleunigung der Tauopathie in Verbindung steht, wird detektiert.

7. Verfahren nach wenigstens einem der Ansprüche 1 bis 6, wobei die Tauopathie die Alzheimer-Krankheit ist.

8. Verwendung eines
(i) diagnostischen Kits umfassend wenigstens ein Reagenz zum Detektieren eines Risikomarkers wie in wenigstens einem der Ansprüche 1 bis 7 beschrieben, oder
(ii) eines Arrays umfassend ein Substrat, das ein oder mehrere Reagenzien zur Identifikation des Vorkommens eines Risikomarkers im HtrA1-Gen in einer Nukleinsäureprobe einer Testperson trägt, wobei der Risikomarker mit dem Risiko der Entwicklung oder Beschleunigung einer Tauopathie in Verbindung steht, wobei der Risikomarker eine Mutation oder ein Polymorphismus des HtrA1-Gens ist,
zur Bestimmung der Anwesenheit oder Abwesenheit wenigstens eines Risikofaktors der Entwicklung oder Beschleunigung einer Tauopathie wie in wenigstens einem der Ansprüche 1 bis 7 beschrieben, wobei die Verwendung zur Durchführung des Verfahrens nach wenigstens einem der Ansprüche 1 bis 7 ist.

9. Verwendung nach Anspruch 8, mit einer oder mehreren der folgenden Eigenschaften:
(i) das Kit umfasst wenigstens einen der folgenden Bestandteile:
a. Primer zum Amplifizieren wenigstens eines Teils des HtrA1-Gens, insbesondere Primer zum Amplifizieren wenigstens eines Teils der Promoterregionen des HtrA1-Gens, die die Stelle des Einzelnukleotidpolymorphismus (SNP) rs11200638 umfasst;
b. wenigstens ein Enzym zur Nukleinsäureamplifikation;
c. wenigstens ein Enzym, das für die Durchführung einer RFLP Analyse zur Identifikation wenigstens eines Risikomarkers geeignet ist;
(ii) das Reagenz des Arrays detektiert den Einzelnukleotidpolymorphismus (SNP) rs11200638 des HtrA1-Gens;
(iii) die Tauopathie ist die Alzheimer-Krankheit.

10. Eine Verbindung, die die HtrA1-Enzymaktivität erhöht, zur Verwendung in einem Medikament zur Proteolyse oder zum Abbau von Tau, phosphoryliertem Tau, hyperphosphoryliertem Tau und/oder Tau-Aggregaten, phosphorylierten Tau-Aggregaten und/oder hyperphosphorylierten Tau-Aggregaten; wobei die Verbindung eine Sequenz umfasst oder aus dieser besteht, ausgewählt aus der Gruppe bestehend aus
a. DQLAFHQFYI
b. XXXDSRIWWV, wobei X eine natürliche oder unnatürliche Aminosäure ist
c. KKKDSRIWWV;
wobei das Medikament zur Behandlung oder zur Prävention einer Tauopathie dient.

11. Verbindung nach Anspruch 10, wobei die Verbindung das Enzym HtrA1 aktiviert oder stabilisiert.

12. In vitro Verwendung von HtrA1 oder einer funktionalen Variante davon die HtrA1-Aktivität aufweist zur Proteolyse oder zum Abbau von Tau, phosphoryliertem Tau, hyperphosphoryliertem Tau und/oder Tau-Aggregaten, phosphorylierten Tau-Aggregaten und/oder hyperphosphorylierten Tau-Aggregaten.

13. Verbindung die die HtrA1-Enzymaktivität erhöht, wie in Anspruch 10 oder 11 definiert, zur Verwendung in der Behandlung oder Prävention der Alzheimer-Krankheit.

14. In vitro Verwendung eines HtrA1-Aktivators zur Förderung der Proteolyse oder des Abbaus von Tau, phosphoryliertem Tau, hyperphosphoryliertem Tau und/oder Tau-Aggregaten, phosphorylierten Tau-Aggregaten und/oder hyperphosphorylierten Tau-Aggregaten; wobei der HtrA1-Aktivator eine Sequenz umfasst oder aus dieser besteht, ausgewählt aus der Gruppe bestehend aus:
a. DQLAFHQFYI
b. XXXDSRIWWV, wobei X eine natürliche oder unnatürliche Aminosäure ist
c. KKKDSRIWWV.

15. Verwendung von HtrA1 in einem Screening-Assay zur Identifizierung einer Verbindung, die die Proteolyse oder den Abbau von Tau, phosphoryliertem Tau, hyperphosphoryliertem Tau und/oder Tau-Aggregaten, phosphorylierten Tau-Aggregaten und/oder hyperphosphorylierten Tau-Aggregaten inhibieren oder fördern kann.

## Revendications

1. Procédé de dépistage chez un sujet d'au moins un facteur de risque associé à une tauopathie, comprenant la détection de la présence ou de l'absence d'au moins un marqueur de risque dans le gène HtrA1 (PRSS11) qui est lié à ladite tauopathie, où le marqueur de risque est un polymorphisme ou une mutation du gène HtrA1.

2. Procédé selon la revendication 1, dans lequel ledit marqueur de risque est au moins une mutation ou un polymorphisme situé dans un élément régulateur du gène HtrA1, en particulier situé dans le promoteur du gène HtrA1.

3. Procédé selon la revendication 2, dans lequel le polymorphisme mononucléotidique (SNP) rs11200638 est détecté.

4. Procédé selon au moins l'une des revendications 1 à 3, dans lequel ladite étape de détection est effectuée en déterminant la présence ou l'absence d'au moins un marqueur de risque dans le gène HtrA1 par analyse de l'acide nucléique dans un échantillon biologique contenant des acides nucléiques recueillis sur ledit patient, où le marqueur de risque, en particulier un polymorphisme, est de préférence détecté par un procédé sélectionné dans le groupe composé des polymorphismes de longueur des fragments de restriction (RFLP), de l'électrophorèse en gel de gradient dénaturant (DGGE), des polymorphismes de conformation des ADN simples brins (SSCP), des analyses par hétéroduplex (HD), un séquençage d'ADN, un pyroséquençage, un typage en utilisant des balises moléculaires, une hybridation dynamique allèle-spécifique (DASH), un système de mutation réfractaire par amplification (ARMS) et une hybridation allèle-spécifique, en particulier sur des puces à ADN, une méthode ELISA.

5. Procédé selon la revendication 4, dans lequel la détection se produit par RFLP en utilisant une enzyme de restriction qui détecte le polymorphisme mononucléotidique (SNP) rs11200638, en particulier qui détecte une séquence d'acides nucléiques comprenant des nucléotides sélectionnés parmi les nucléotides CGGCCG pour identifier l'allèle HtrA1 G marqué, en particulier EagI, BsiEI, EaeI, GdiII, Sfil et BgII et leurs isoschizomères respectifs, ou qui détecte une séquence d'acides nucléiques comprenant des nucléotides sélectionnés parmi les nucléotides CAGCCG pour identifier l'allèle HtrA1 A marqué ; le procédé comprenant de préférence les étapes suivantes:
a. amplification d'au moins une partie du gène HtrA 1 de l'échantillon du sujet en utilisant des amorces oligonucléotidiques appropriées,
b. incubation du dit acide nucléique amplifié avec une enzyme de restriction appropriée pour détecter le marqueur de risque respectif, en particulier le polymorphisme mononucléotidique (SNP) rs11200638,
c. détermination de la taille des fragments de restriction obtenus, analysant de ce fait si le sujet porte un facteur de risque de ladite tauopathie, notamment le risque de l'âge d'apparition (AAO).

6. Procédé selon au moins l'une des revendications 1 à 5 précédentes, présentant une ou plusieurs des caractéristiques suivantes:
(i) les amorces oligonucléotidiques suivantes sont utilisées pour l'amplification:
amorce sens: 5'-ATGCCACCCACAACAACTTT-3' ;
amorce antisens: 5'-CGCGTCCTTCAAACTAATGG-3'
(ii) la présence ou l'absence d'au moins un ou plusieurs marqueurs de risque supplémentaires liés à ladite tauopathie est détectée, où ledit marqueur de risque supplémentaire, au moins au nombre de un, détecte de préférence la présence ou l'absence d'au moins une mutation ou un polymorphisme au sein du gène ApoE;
(iii)l'occurrence d'un variant allélique associé au risque de développer ou d'accélérer ladite tauopathie est détectée.

7. Procédé selon au moins l'une des revendications 1 à 6, dans lequel ladite tauopathie est la maladie d'Alzheimer.

8. Utilisation
(i) d'un kit de diagnostic comprenant au moins un réactif permettant de détecter un marqueur de risque tel que décrit dans au moins l'une des revendications 1 à 7, ou
(ii) d'une matrice comprenant un substrat qui porte un ou plusieurs réactifs permettant d'identifier dans un échantillon d'acide nucléique d'un sujet l'occurrence d'un marqueur de risque dans le gène HtrA1 qui est associé au risque de développer ou d'accélérer une tauopathie, où le marqueur de risque est une mutation ou un polymorphisme du gène HtrA 1,
afin de déterminer la présence ou l'absence d'au moins un facteur de risque, tel que décrit dans au moins l'une des revendications 1 à 7, de développer ou d'accélérer une tauopathie, où l'utilisation vise à réaliser le procédé selon au moins l'une des revendications 1 à 7.

9. Utilisation selon la revendication 8, présentant une ou plusieurs des caractéristiques suivantes:
(i) le kit comprend au moins l'un des composants suivants:
a. amorces pour amplifier au moins une partie du gène HtrA 1, en particulier des amorces pour amplifier au moins une partie de la région promoteur du gène HtrA1 englobant le site du polymorphisme mononucléotidique (SNP) rs11200638;
b. au moins une enzyme pour l'amplification de l'acide nucléique;
c. au moins une enzyme appropriée pour réaliser une analyse de RFLP permettant d'identifier au moins un marqueur de risque;
(ii) ledit réactif de la matrice détecte le polymorphisme mononucléotidique (SNP) rs11200638 du gène HtrA1;
(iii)ladite tauopathie est la maladie d'Alzheimer.

10. Composé améliorant l'activité de l'enzyme HtrA1 à utiliser dans un médicament destiné à la protéolyse ou à la dégradation de tau, de tau phosphorylé, de tau hyperphosphorylé et/ou d'agrégats de tau, d'agrégats de tau phosphorylés et/ou d'agrégats de tau hyperphosphorylés; où le composé comprend ou se compose d'une séquence sélectionnée dans le groupe composé de:
a. DQLAFHQFYI
b. XXXDSRIWWV, où X est un acide aminé naturel ou non naturel
c. KKKDSRIWWV,
où le médicament est destiné au traitement ou à la prévention d'une tauopathie.

11. Composé selon la revendication 10, dans lequel ledit composé active ou stabilise l'enzyme HtrA1.

12. Utilisation in vitro de HtrA1 ou d'un variant fonctionnel de celui-ci ayant une activité de HtrAl pour la protéolyse ou la dégradation de tau, de tau phosphorylé, de tau hyperphosphorylé et/ou d'agrégats de tau, d'agrégats de tau phosphorylés et/ou d'agrégats de tau hyperphosphorylés.

13. Composé améliorant l'activité de l'enzyme HtrA1 tel que défini dans la revendication 10 ou 11, à utiliser dans le traitement ou la prévention de la maladie d'Alzheimer.

14. Utilisation in vitro d'un activateur de HtrA1 afin de promouvoir la protéolyse ou la dégradation de tau, de tau phosphorylé, de tau hyperphosphorylé et/ou d'agrégats de tau, d'agrégats de tau phosphorylés et/ou d'agrégats de tau hyperphosphorylés; où l'activateur d'HtrA1 comprend ou se compose de la séquence sélectionnée dans le groupe composé de:
a. DQLAFHQFYI
b. XXXDSRIWWV, où X est un acide aminé naturel ou non naturel
c. KKKDSRIWWV.

15. Utilisation de HtrA1 dans un essai de dépistage permettant d'identifier un composé capable d'inhiber ou de promouvoir la protéolyse ou la dégradation de tau, de tau phosphorylé, de tau hyperphosphorylé et/ou d'agrégats de tau, d'agrégats de tau phosphorylés et/ou d'agrégats de tau hyperphosphorylés.
